# EUROPEAN PATENT APPLICATION

(11) **EP 4 725 956 A1**
(43) Date of publication of application: **15.04.2026**
(21) Application number: 24824881.7
(22) Date of filing: 24.01.2024
(51) Int. Cl.: C07K 7/08, A61K 38/10, A61P 25/04

(54) **MODIFIED POLYPEPTIDE AND USE THEREOF IN FIELD OF ANALGESIA**

(30) Priority: 21.06.2023 WO PCT/CN2023/101880
(71) Applicant: Nanjing Anji Biotechnology Co., Ltd., Nanjing, Jiangsu 210000 (CN)
(72) Inventor: XU, Hanmei, Nanjing, Jiangsu 210000 (CN); ZHOU, Tianxiong, Nanjing, Jiangsu 210000 (CN)
(74) Representative: Prüfer & Partner mbB Patentanwälte · Rechtsanwälte
(86) International application number: PCT/CN2024/073896
(87) International publication number: WO 2024/259977

(57) **Abstract**

The present application relates to a peptide or a derivative of the peptide, a pharmaceutical composition containing the peptide or the derivative of the peptide, and the use thereof in the treatment of pain.

## Description

### CROSS-REFERENCE TO RELATED APPLICATIONS

This application claims priority to PCT/CN2023/101880 filed on June 21, 2023. The earlier application is considered part of the disclosure of the present application and is incorporated herein in its entirety.

### TECHNICAL FIELD

The present application relates to the field of biomedicine, and in particular, to a peptide or a derivative thereof and its use in analgesia.

### BACKGROUND

Neuropathic pain refers to pain caused by lesions or diseases of the somatosensory nervous system. It is a common type of chronic pain that seriously affects patients' quality of life. It is estimated that about 8% of the global population suffers from neuropathic pain (GILRON I, BARON R, JENSEN T. Neuropathic pain: principles of diagnosis and treatment [J]. Mayo Clin Proc, 2015, 90(4): 532-545). Metabolic disorders (e.g., diabetic neuropathy), nerve entrapment, tumor-related pain, post-stroke pain, Parkinson's disease-related pain, post-spinal cord injury pain, trigeminal neuralgia, glossopharyngeal neuralgia, and viral infections such as postherpetic neuralgia are all typical examples of neuropathic pain (SZOK D, TAJTI J, NYÁRI A, et al. Therapeutic approaches for peripheral and central neuropathic pain [J]. Behav Neurol, 2019, 2019: 8685954; Jensen TS, Baron R, Haanp M, et al. A new definition of neuropathic pain [J]. Pain, 2011, 152(10): 2204-2205).

Peripheral neuropathy is characterized by nerve injury and axonal loss, which may be of genetic or acquired origin. Acquired peripheral neuropathy is associated with multiple causes, including exposure to toxic agents, among which pain induced by antineoplastic compounds is referred to as chemotherapy-induced peripheral neuropathy (CIPN). A review has summarized several preclinical studies on CIPN, highlighting neuroimmune interactions that indicate a link between chemotherapeutic agents and neurotoxicity. Studies have demonstrated that both innate and adaptive immune responses, along with the stimulation and secretion of mediators such as cytokines and chemokines, may contribute to pain symptoms. Furthermore, neuroinflammatory components have been observed in the spinal cord, with microglia and astrocytes playing important roles in CIPN (Front Immunol., 2021 Feb 4; 11:626687).

Nicotinic acetylcholine receptors (nAChRs) are membrane proteins widely present in nature that play essential physiological roles, regulating various biological functions such as pain perception, cognition, memory, and anxiety. Acetylcholine receptors are widely distributed in neurons and are closely associated with analgesia. The main receptor subtypes are α7 and α9α10 (see, for example, Hone A.J. et al., Nicotinic acetylcholine receptors in neuropathic and inflammatory pain, FEBS Lett., 2018 Apr; 592(7): 1045-1062; Wu Pei et al., Role of α7 nicotinic acetylcholine receptor in regulating inflammatory responses in animals, Acta Zoonutrimenta Sinica, 2021, 33(11): 6001-6008). Cone snails are carnivorous mollusks belonging to the family Conidae, comprising approximately 700 species distributed mainly in tropical seas. Conotoxins are neurotoxic peptides secreted by cone snails, consisting of 10-30 amino acids and comprising disulfide bonds. The research team led by Professor Olivera at the University of Utah isolated and identified numerous conotoxin sequences and their biological activities, discovering that conotoxins could serve as potential therapeutic agents for various neurological disorders. This discovery has spurred worldwide research into the development, structural modification, and mechanism of action of conotoxins. Vc1.1, also known as ACV1, is a 16-amino acid peptide with two pairs of disulfide bonds, which was isolated from Conus victoriae. It is an α-conotoxin with analgesic activity and acts as a selective antagonist of the α9α10 nicotinic acetylcholine receptor (nAChR). In rat models of chronic constriction injury (CCI) of the sciatic nerve and neuropathic pain (PNL), Vc1.1 demonstrated therapeutic efficacy against neuropathic pain. However, due to differences between human and rat α9α10 nAChRs, the activity of Vc1.1 on human receptors was reduced by approximately 100-fold compared to rat receptors. As a result, although Vc1.1 entered phase II clinical trials, the significant loss of potency in humans led to the discontinuation of the clinical study. Lorenzo Di Cesare Mannelli et al. investigated the analgesic effect and mechanism of the conotoxin peptide RgIA in a rat chronic constriction injury (CCI) model of sciatic nerve ligation. Histological analysis of the sciatic nerve showed that RgIA prevented CCI-induced axonal compaction, diameter reduction, myelin loss, and fiber decrease. Moreover, RgIA significantly reduced edema and inflammatory infiltration, including a decrease in CD86+ macrophages. In the dorsal horn of the spinal cord, RgIA inhibited CCI-induced activation of microglia and astrocytes. These data suggest that peptide RgIA may represent a new class of drugs for the treatment of neuropathic pain, capable of protecting peripheral nerve tissue and preventing maladaptive central plasticity by suppressing glial cell activation (PAIN, 155 (2014): 1986-1995).

Ziconotide was approved by the FDA in 2004 for the treatment of severe chronic pain. It was originally isolated from the venom of Conus magus and comprises 25 amino acids with three pairs of disulfide bonds. As a selective Cav2.2 channel antagonist, ziconotide blocks Ca²⁺ influx and inhibits the release of excitatory neurotransmitters from afferent nerve terminals. Its analgesic potency is approximately 1000 times that of opioids. However, the intrathecal route of administration, coupled with a range of central nervous system adverse effects, leads to patient compliance, thereby limiting the widespread clinical use of ziconotide.

Therefore, there is an urgent need to develop an improved peptide that targets nicotinic acetylcholine receptors and can effectively relieve pain and/or reduce adverse effects.

### SUMMARY

The peptides disclosed in the present application target α7 nAChR and α9 nAChR, which are acetylcholine receptors of human origin. This design avoids the activity reduction and similar risks caused by species differences observed with the conotoxin Vc1.1. The peptide sequences of the present application incorporate multiple non-natural amino acids, and the peptide structure comprises one pair of intramolecular disulfide bonds. These features enhance the stability of the peptide, significantly increase its in vivo half-life, and markedly extend the duration of its analgesic effect. Pain is a serious clinical problem that adversely affects the quality of life of both patients and their families. However, existing analgesics cannot specifically address different types of pain and often cause adverse effects with long-term use, such as the addiction potential of opioid drugs and the associated risk of abuse. Peptides, by contrast, possess inherent safety advantages. The peptides disclosed herein have simple synthetic processes, are suitable for commercial-scale production, and are expected to serve as a new class of safe and effective analgesic agents.

According to animal experiment results, the peptides of the present application show a faster onset of action than the clinically used analgesic pregabalin. Moreover, compared with the parent peptide AJ003, the peptides disclosed herein exhibit significantly improved stability in human plasma. Animal data further demonstrate that the modified peptides show a markedly prolonged duration of analgesic efficacy compared to the parent peptide. The peptides of the present application are novel non-opioid analgesics. Because their target sites are mainly located in peripheral nerve tissues, they are unlikely to cause drug addiction or other toxic side effects in clinical use. Compared with ziconotide-the only conotoxin peptide currently approved for marketing-the peptides of the present application do not require the complex intrathecal infusion route of administration, which improves patient compliance in clinical treatment. Furthermore, ziconotide is composed of 25 amino acids and comprises three pairs of disulfide bonds, making its preparation process complex and costly. In contrast, the peptides of the present application are composed of 13 amino acids and comprise only one pair of disulfide bonds. Their preparation process is simple, and the solid-phase peptide synthesis (SPPS) technology is now well established, which is highly advantageous for automated large-scale production. This greatly reduces production costs and eases the financial burden on patients. In addition, the peptides disclosed herein are effective at low doses, exhibiting significant analgesic activity even at low dosage levels.

In a first aspect of the present disclosure, there is provided a peptide or a derivative thereof comprising any one of the sequences selected from SEQ ID NOs: 1-34.

In a second aspect of the present disclosure, there is provided a peptide or a derivative thereof comprising any one of the sequences selected from SEQ ID NOs: 1-34 having conservative substitutions.

In a third aspect of the present disclosure, there is provided a peptide or a derivative thereof comprising, in order from the N-terminus to the C-terminus, the sequence of Formula I or a conservatively substituted variant thereof:

Gly-Ser-Cys-Ser-X1-X2-X3-X4-(D-Cys)-X5-X6-X7-X8 Formula I

In Formula I, the Cys residue at position 3 and the D-Cys residue at position 9 form one intramolecular disulfide bond. D-Cys represents D-cysteine, and the "-" symbol between amino acid residues represents a peptide bond. The C-terminus of the peptide or a derivative thereof is a free carboxyl or amide. X1 is Thr or D-Thr, X5 is any one selected from Ala, Val, and D-Val, and X2-X4 and X6-X8 are each independently any amino acid.

In an example of the present disclosure, the Cys residue at position 3 and the D-Cys residue at position 9 in Formula I form one intramolecular disulfide bond. The C-terminus of the peptide or a derivative thereof is a free carboxyl or amide. X1 is Thr or D-Thr; X5 is any one selected from Ala, Val, and D-Val; X2 is any one selected from Pro, D-Pro, and Hyp; X3 is any one selected from Pro, D-Pro, and Hyp; X4 is any one selected from Ser, D-Ser, Ala, and Aib; X6 is any one selected from Ala, Leu, D-Leu, Ile, ABu, and Nle; X7 is any one selected from Tyr, D-Tyr, Ala, Trp, Phe, and Bip; and X8 is any one selected from Ser, D-Ser, Ala, and Orn.

In an example of the present disclosure, the conservatively substituted variant of Formula I has 1, 2, 3, 4, or 5 amino acid conservative substitutions. In an example of the present disclosure, the conservatively substituted variant of Formula I has at least 65%, 70%, 75%, 80%, 84%, 85%, 90%, 92%, or 95% sequence identity with the unsubstituted Formula I sequence.

In a preferred example of the present disclosure, the peptide or a derivative thereof comprises any one sequence selected from the following sequences: SEQ ID NOs: 1-5, SEQ ID NOs: 10-16, and SEQ ID NOs: 20-34, or a sequence thereof having conservative substitutions. In a more preferred example of the present disclosure, the peptide or a derivative thereof comprises any one sequence selected from the following sequences: SEQ ID NOs: 26, 28, and 32, or a sequence thereof having conservative substitutions.

In an example of the present disclosure, the conservatively substituted sequences described above each comprise 1, 2, 3, 4, or 5 conservative amino acid substitutions. In an example of the present disclosure, the conservatively substituted sequences described above have at least 65%, at least 70%, at least 75%, at least 80%, at least 84%, at least 85%, at least 90%, at least 92%, or at least 95% sequence identity with the corresponding sequences without conservative substitutions.

In a fourth aspect of the present disclosure, there is provided a polynucleotide encoding the peptide or a derivative thereof disclosed herein.

In a fifth aspect of the present disclosure, there is provided an expression vector expressing the polynucleotide disclosed herein.

In a sixth aspect of the present disclosure, there is provided a host cell comprising the polynucleotide or expression vector disclosed herein.

In a seventh aspect of the present disclosure, there is provided a pharmaceutical composition comprising the peptide or a derivative thereof disclosed herein, and a pharmaceutically acceptable carrier or salt thereof. The present disclosure further provides a pharmaceutical composition comprising the peptide or a derivative thereof, the polynucleotide, the expression vector, or the host cell disclosed herein, together with a pharmaceutically acceptable carrier or salt thereof.

In an eighth aspect of the present disclosure, there is provided the use of the peptide or a derivative thereof, or the pharmaceutical composition disclosed herein, in the preparation of a medicament for treating pain. The present disclosure further provides the use of the peptide or a derivative thereof, the polynucleotide, the expression vector, the host cell, or the pharmaceutical composition disclosed herein, in the preparation of a medicament for treating pain.

In a ninth aspect of the present disclosure, there is provided a method for treating pain, comprising administering to a subject in need thereof a therapeutically effective amount of the peptide or a derivative thereof, or the pharmaceutical composition disclosed herein. The present disclosure further provides a method for treating pain, comprising administering to a subject in need thereof a therapeutically effective amount of the peptide or a derivative thereof, the polynucleotide, the expression vector, the host cell, or the pharmaceutical composition disclosed herein.

### BRIEF DESCRIPTION OF THE DRAWINGS

The following provides a brief description of the drawings, which are intended to illustrate exemplary embodiments disclosed herein and are not to be construed as limiting the embodiments.

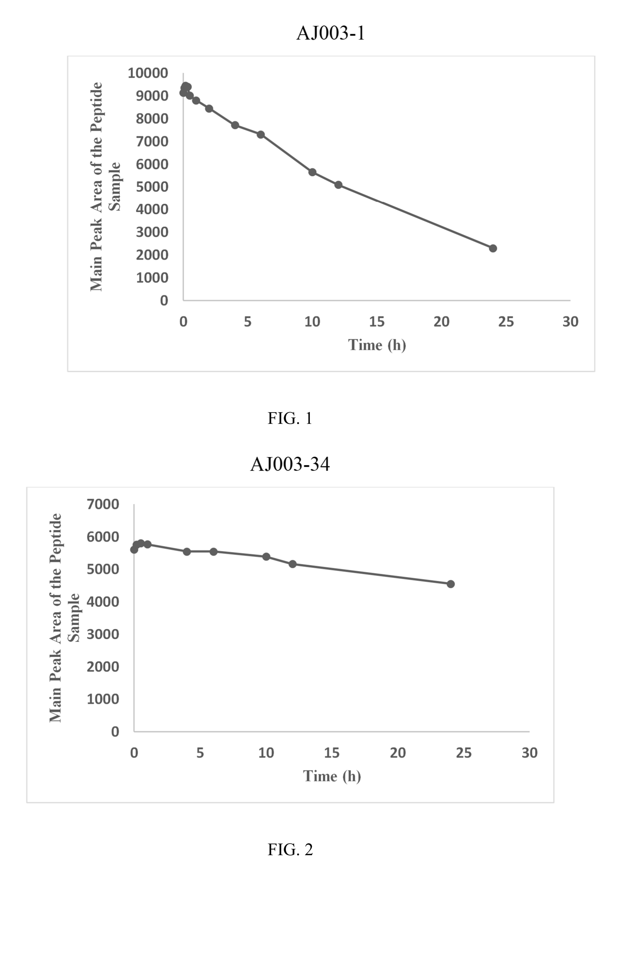

FIG. 1 shows a line graph illustrating the change in the main peak area of peptide AJ003-1 over time in Example 3.
FIG. 2 shows a line graph illustrating the change in the main peak area of peptide AJ003-34 over time in Example 3.

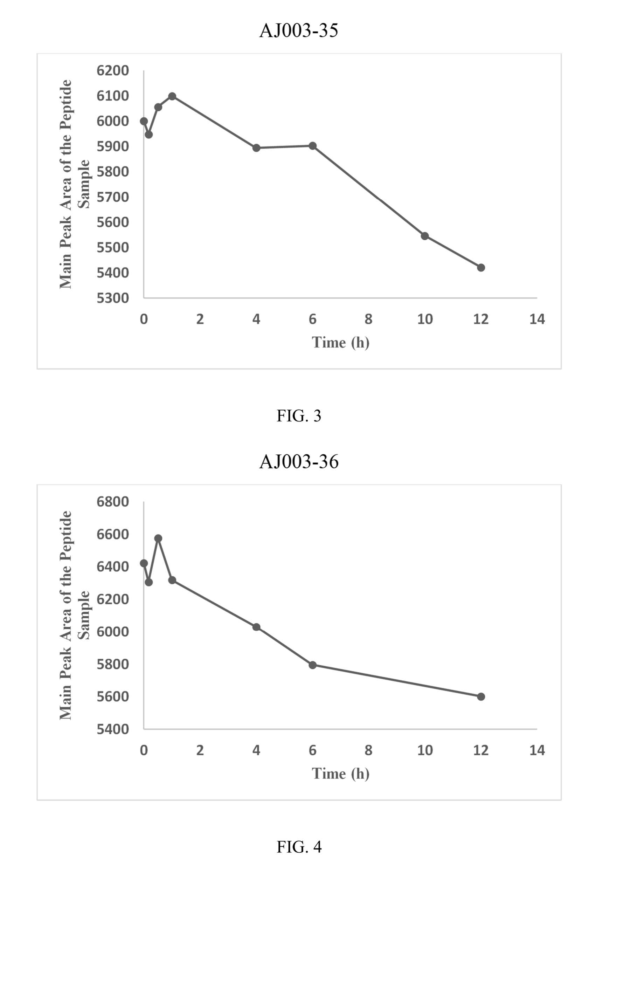

FIG. 3 shows a line graph illustrating the change in the main peak area of peptide AJ003-35 over time in Example 3.
FIG. 4 shows a line graph illustrating the change in the main peak area of peptide AJ003-36 over time in Example 3.

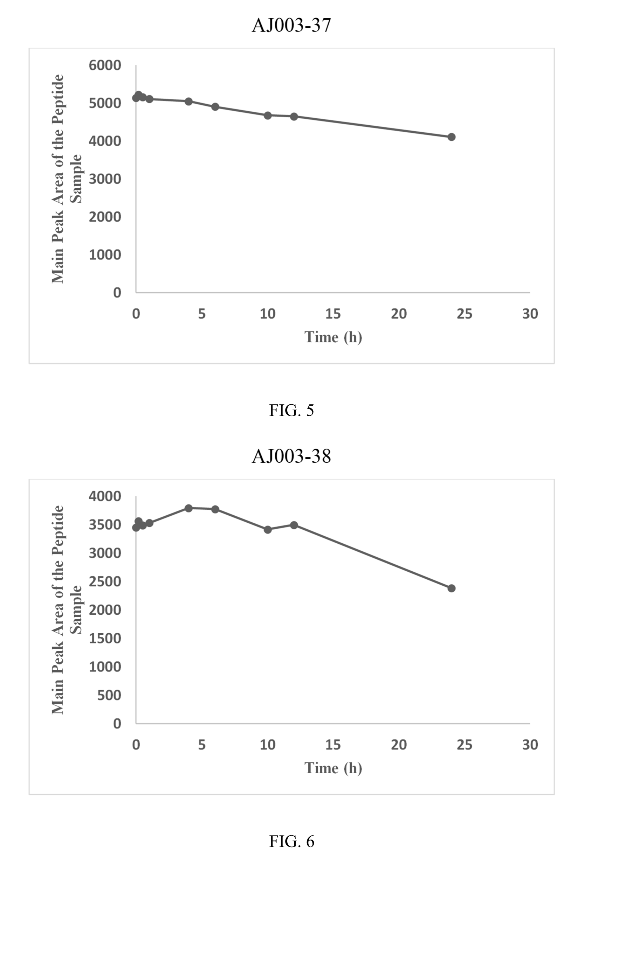

FIG. 5 shows a line graph illustrating the change in the main peak area of peptide AJ003-37 over time in Example 3.
FIG. 6 shows a line graph illustrating the change in the main peak area of peptide AJ003-38 over time in Example 3.

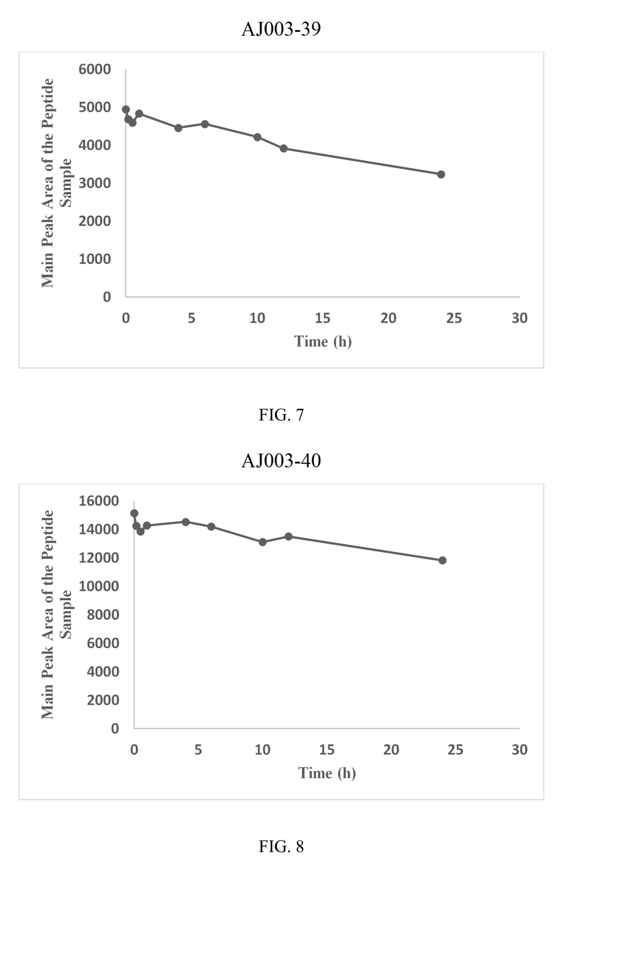

FIG. 7 shows the predicted in vitro half-life result of peptide AJ003-39 in human plasma in Example 3.
FIG. 8 shows a line graph illustrating the change in the main peak area of peptide AJ003-40 over time in Example 3.

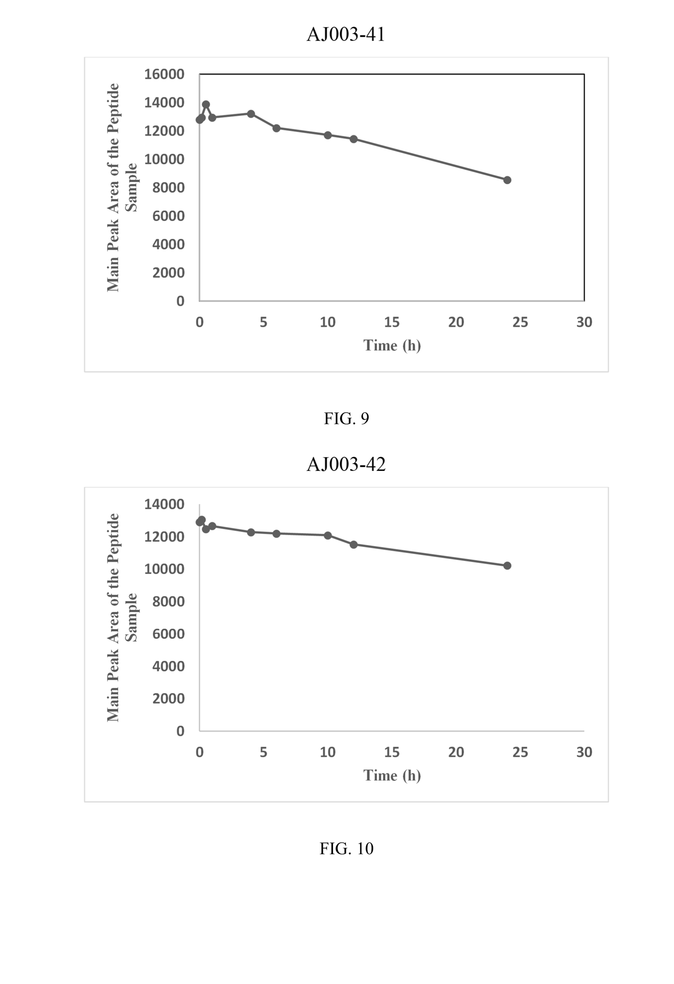

FIG. 9 shows a line graph illustrating the change in the main peak area of peptide AJ003-41 over time in Example 3.
FIG. 10 shows a line graph illustrating the change in the main peak area of peptide AJ003-42 over time in Example 3.

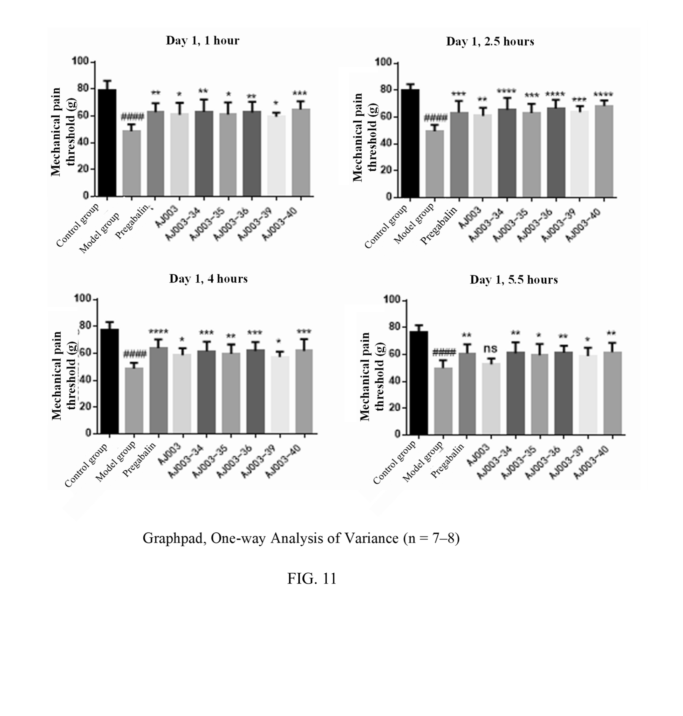

FIG. 11 shows the mechanical pain threshold on Day 1 in the rat paclitaxel-induced chronic chemotherapy pain model after administration of peptides at the same dose in Example 5. The vertical axis represents the mechanical pain threshold, and the horizontal axis represents different groups. The results are expressed as mechanical pain threshold (g) (±SD).Statistical analysis was performed using GraphPad and one-way ANOVA (n = 7-8). Model group vs. control group: #### p < 0.0001;Drug-treated groups vs. model group: **** p < 0.0001; *** p < 0.001; ** p < 0.01; * p < 0.05; ns: not statistically significant. Pregabalin was administered at 30 mg/kg·d·qd via oral gavage. AJ003, AJ003-34, AJ003-35, AJ003-36, AJ003-39, and AJ003-40 were administered at 1 mg/kg·d·qd via subcutaneous injection.

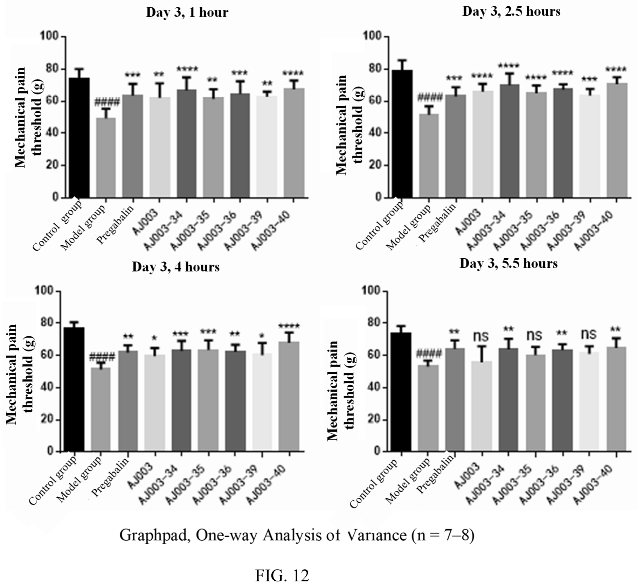

FIG. 12 shows the mechanical pain threshold on Day 3 in the rat paclitaxel-induced chronic chemotherapy pain model after administration of peptides at the same dose in Example 5. The vertical axis represents the mechanical pain threshold, and the horizontal axis represents different groups. The results are expressed as mechanical pain threshold (g) (±SD). Statistical analysis was performed using GraphPad and one-way ANOVA (n = 7-8). Model group vs. control group: #### p < 0.0001; Drug-treated groups vs. model group: **** p < 0.0001; *** p < 0.001; ** p < 0.01; * p < 0.05; ns: not statistically significant. Pregabalin was administered at 30 mg/kg·d·qd via oral gavage. AJ003, AJ003-34, AJ003-35, AJ003-36, AJ003-39, and AJ003-40 were administered at 1 mg/kg·d·qd via subcutaneous injection.

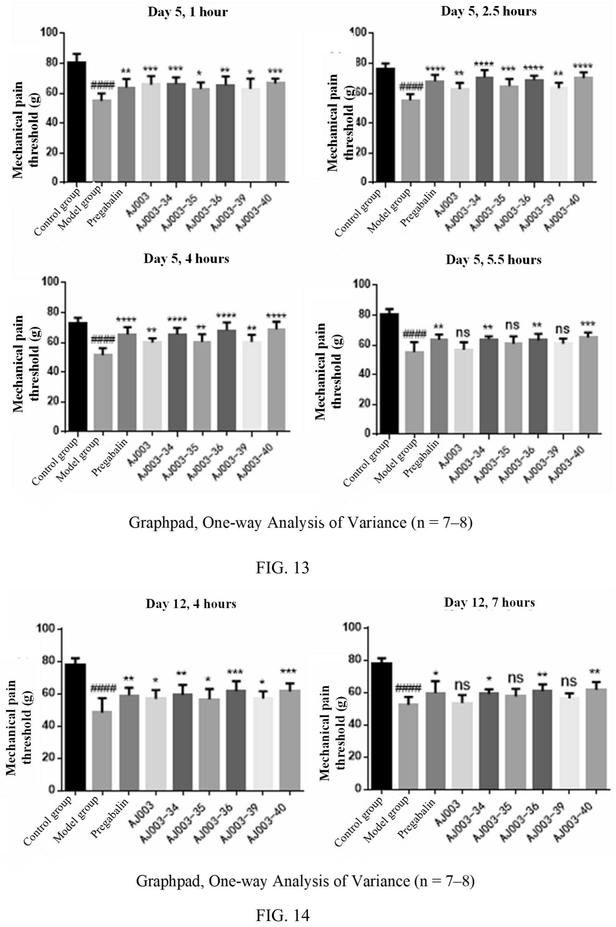

FIG. 13 shows the mechanical pain threshold on Day 5 in the rat paclitaxel-induced chronic chemotherapy pain model after administration of peptides at the same dose in Example 5. The vertical axis represents the mechanical pain threshold, and the horizontal axis represents different groups. The results are expressed as mechanical pain threshold (g) (±SD). Statistical analysis was performed using GraphPad and one-way ANOVA (n = 7-8). Model group vs. control group: #### p < 0.0001; Drug-treated groups vs. model group: **** p < 0.0001; *** p < 0.001; ** p < 0.01; * p < 0.05; ns: not statistically significant. Pregabalin was administered at 30 mg/kg·d·qd via oral gavage. AJ003, AJ003-34, AJ003-35, AJ003-36, AJ003-39, and AJ003-40 were administered at 1 mg/kg·d·qd via subcutaneous injection.
FIG. 14 shows the mechanical pain threshold on Day 12 in the rat paclitaxel-induced chronic chemotherapy pain model after administration of peptides at the same dose in Example 5. The vertical axis represents the mechanical pain threshold, and the horizontal axis represents different groups. The results are expressed as mechanical pain threshold (g) (±SD).Statistical analysis was performed using GraphPad and one-way ANOVA (n = 7-8). Model group vs. control group: #### p < 0.0001; Drug-treated groups vs. model group: **** p < 0.0001; *** p < 0.001; ** p < 0.01; * p < 0.05; ns: not statistically significant. Pregabalin was administered at 30 mg/kg·d·qd via oral gavage. AJ003, AJ003-34, AJ003-35, AJ003-36, AJ003-39, and AJ003-40 were administered at 1 mg/kg·d·qd via subcutaneous injection.

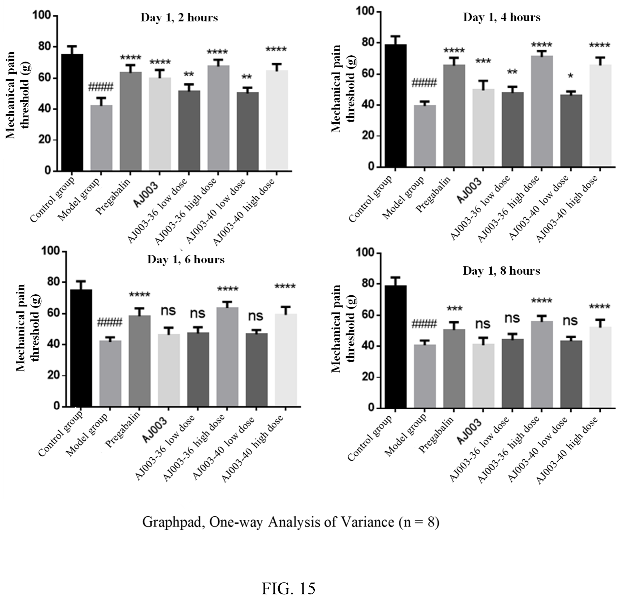

FIG. 15 shows the mechanical pain threshold on Day 1 in the rat paclitaxel-induced chronic chemotherapy pain model after administration of peptides at different doses in Example 6. The vertical axis represents the mechanical pain threshold, and the horizontal axis represents different groups. The results are expressed as mechanical pain threshold (g) (±SD). Statistical analysis was performed using GraphPad and one-way ANOVA (n = 8). Model group vs. control group: #### p < 0.0001; Drug-treated groups vs. model group: **** p < 0.0001; *** p < 0.001; ** p < 0.01; * p < 0.05; ns: not statistically significant. Pregabalin was administered at 30 mg/kg·d·qd via oral gavage. AJ003 was administered at 1 mg/kg·d·qd via subcutaneous injection. AJ003-36 (low dose) was administered at 0.5 mg/kg·d·qd via subcutaneous injection. AJ003-36 (high dose) was administered at 1.5 mg/kg·d·qd via subcutaneous injection. AJ003-40 (low dose) was administered at 0.5 mg/kg·d·qd via subcutaneous injection. AJ003-40 (high dose) was administered at 1.5 mg/kg·d·qd via subcutaneous injection.

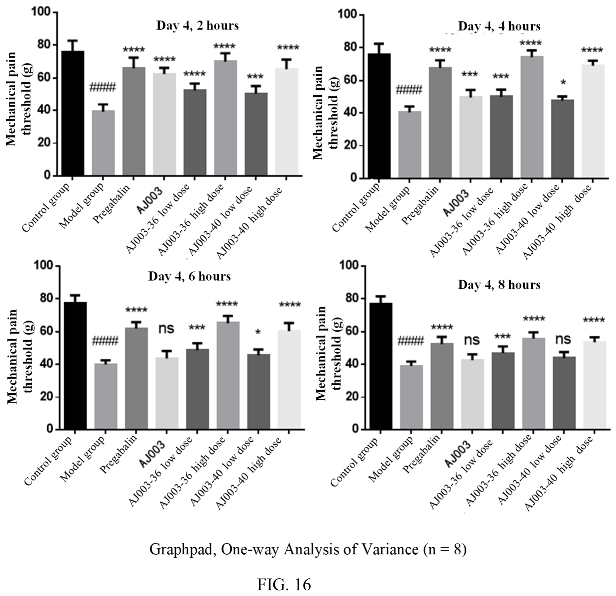

FIG. 16 shows the mechanical pain threshold on Day 4 in the rat paclitaxel-induced chronic chemotherapy pain model after administration of peptides at different doses in Example 6. The vertical axis represents the mechanical pain threshold, and the horizontal axis represents different groups. The results are expressed as mechanical pain threshold (g) (±SD). Statistical analysis was performed using GraphPad and one-way ANOVA (n = 8). Model group vs. control group: #### p < 0.0001; Drug-treated groups vs. model group: **** p < 0.0001; *** p < 0.001; ** p < 0.01; * p < 0.05; ns: not statistically significant. Pregabalin was administered at 30 mg/kg·d·qd via oral gavage. AJ003 was administered at 1 mg/kg·d·qd via subcutaneous injection. AJ003-36 (low dose) was administered at 0.5 mg/kg·d·qd via subcutaneous injection. AJ003-36 (high dose) was administered at 1.5 mg/kg·d·qd via subcutaneous injection. AJ003-40 (low dose) was administered at 0.5 mg/kg·d·qd via subcutaneous injection. AJ003-40 (high dose) was administered at 1.5 mg/kg·d·qd via subcutaneous injection.

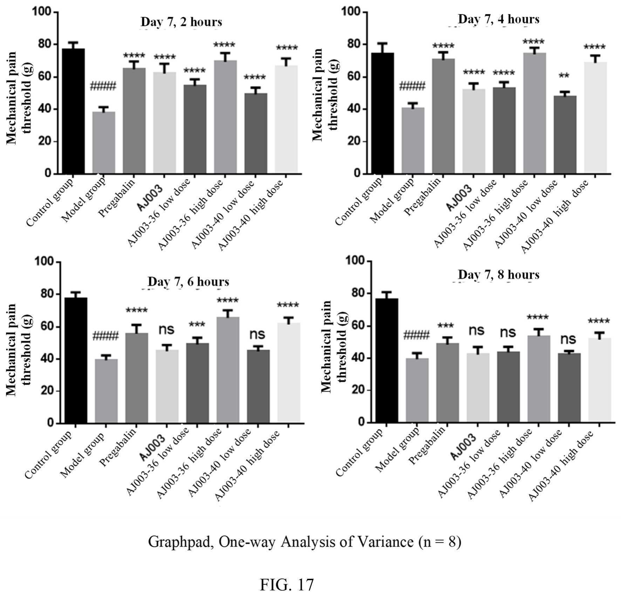

FIG. 17 shows the mechanical pain threshold on Day 7 in the rat paclitaxel-induced chronic chemotherapy pain model after administration of peptides at different doses in Example 6. The vertical axis represents the mechanical pain threshold, and the horizontal axis represents different groups. The results are expressed as mechanical pain threshold (g) (±SD). Statistical analysis was performed using GraphPad and one-way ANOVA (n = 8). Model group vs. control group: #### p < 0.0001; Drug-treated groups vs. model group: **** p < 0.0001; *** p < 0.001; ** p < 0.01; * p < 0.05; ns: not statistically significant. Pregabalin was administered at 30 mg/kg·d·qd via oral gavage. AJ003 was administered at 1 mg/kg·d·qd via subcutaneous injection. AJ003-36 (low dose) was administered at 0.5 mg/kg·d·qd via subcutaneous injection. AJ003-36 (high dose) was administered at 1.5 mg/kg·d·qd via subcutaneous injection. AJ003-40 (low dose) was administered at 0.5 mg/kg·d·qd via subcutaneous injection. AJ003-40 (high dose) was administered at 1.5 mg/kg·d·qd via subcutaneous injection.

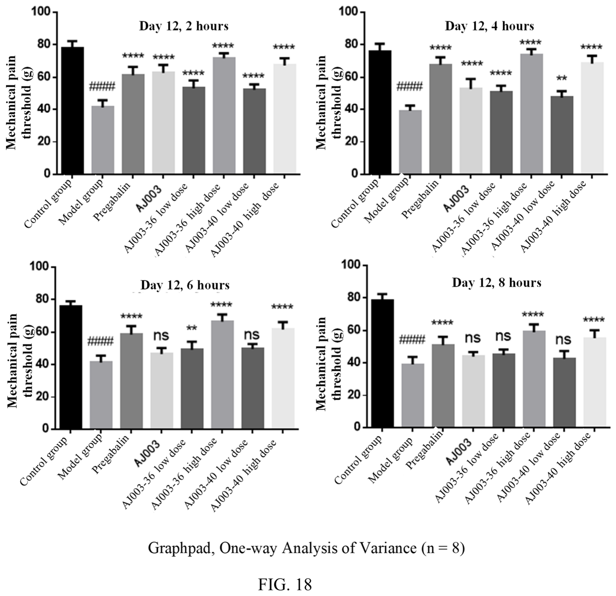

FIG. 18 shows the mechanical pain threshold on Day 12 in the rat paclitaxel-induced chronic chemotherapy pain model after administration of peptides at different doses in Example 6. The vertical axis represents the mechanical pain threshold, and the horizontal axis represents different groups. The results are expressed as mechanical pain threshold (g) (±SD). Statistical analysis was performed using GraphPad and one-way ANOVA (n = 8). Model group vs. control group: #### p < 0.0001; Drug-treated groups vs. model group: **** p < 0.0001; *** p < 0.001; ** p < 0.01; * p < 0.05; ns: not statistically significant. Pregabalin was administered at 30 mg/kg·d·qd via oral gavage. AJ003 was administered at 1 mg/kg·d·qd via subcutaneous injection. AJ003-36 (low dose) was administered at 0.5 mg/kg·d·qd via subcutaneous injection. AJ003-36 (high dose) was administered at 1.5 mg/kg·d·qd via subcutaneous injection. AJ003-40 (low dose) was administered at 0.5 mg/kg·d·qd via subcutaneous injection. AJ003-40 (high dose) was administered at 1.5 mg/kg·d·qd via subcutaneous injection.

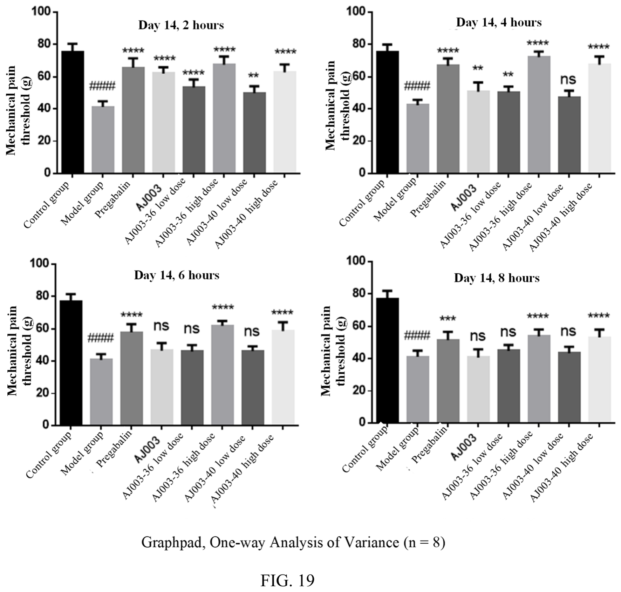

FIG. 19 shows the mechanical pain threshold on Day 14 in the rat paclitaxel-induced chronic chemotherapy pain model after administration of peptides at different doses in Example 6. The vertical axis represents the mechanical pain threshold, and the horizontal axis represents different groups. The results are expressed as mechanical pain threshold (g) (±SD). Statistical analysis was performed using GraphPad and one-way ANOVA (n = 8). Model group vs. control group: #### p < 0.0001; Drug-treated groups vs. model group: **** p < 0.0001; *** p < 0.001; ** p < 0.01; * p < 0.05; ns: not statistically significant. Pregabalin was administered at 30 mg/kg·d·qd via oral gavage. AJ003 was administered at 1 mg/kg·d·qd via subcutaneous injection. AJ003-36 (low dose) was administered at 0.5 mg/kg·d·qd via subcutaneous injection. AJ003-36 (high dose) was administered at 1.5 mg/kg·d·qd via subcutaneous injection. AJ003-40 (low dose) was administered at 0.5 mg/kg·d·qd via subcutaneous injection. AJ003-40 (high dose) was administered at 1.5 mg/kg·d·qd via subcutaneous injection.

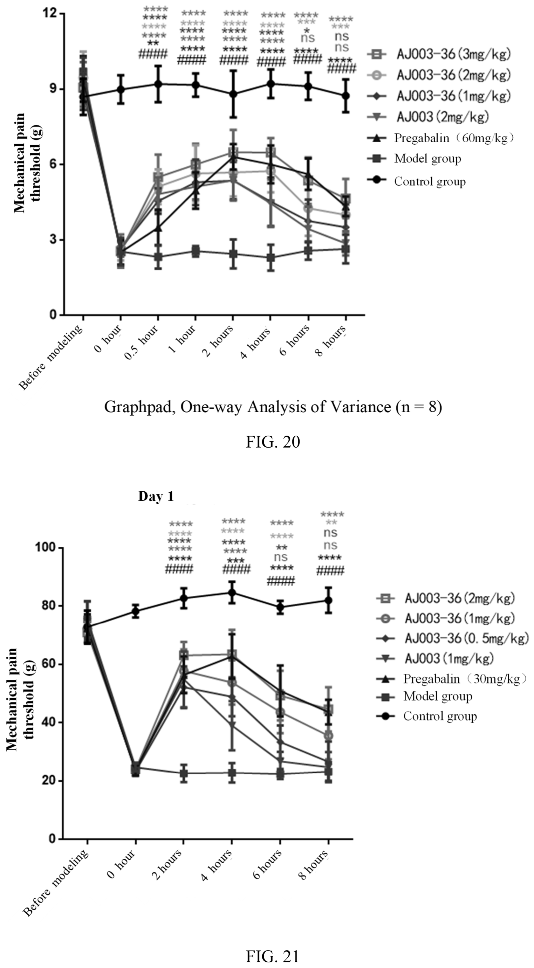

FIG. 20 shows the time-dependent changes in the mechanical pain threshold in the mouse oxaliplatin-induced acute chemotherapy pain model after administration of peptides at different doses in Example 7. The results are expressed as mechanical pain threshold (g) (±SD). Statistical analysis was performed using GraphPad and one-way ANOVA (n = 8). Model group vs. control group: #### p < 0.0001; Drug-treated groups vs. model group: **** p < 0.0001; *** p < 0.001; ** p < 0.01; * p < 0.05; ns: not statistically significant.
FIG. 21 shows the mechanical pain threshold on Day 1 in the rat CCI (chronic constriction injury) model after administration of peptides at different doses in Example 9. The results are expressed as mechanical pain threshold (g) (±SD). Statistical analysis was performed using GraphPad and one-way ANOVA (n = 7). Model group vs. control group: #### p < 0.0001; Drug-treated groups vs. model group: **** p < 0.0001; *** p < 0.001; ** p < 0.01; * p < 0.05; ns: not statistically significant.

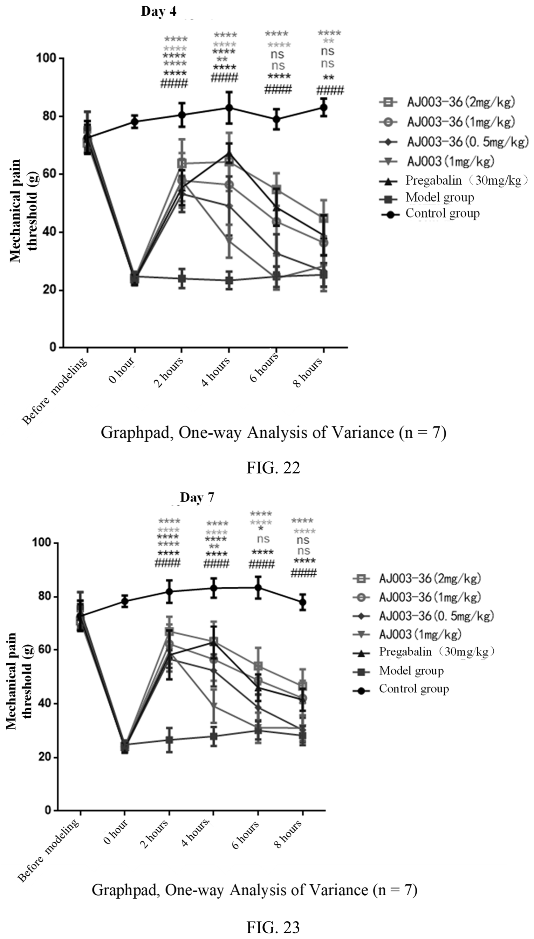

FIG. 22 shows the mechanical pain threshold on Day 4 in the rat CCI model after administration of peptides at different doses in Example 9. The results are expressed as mechanical pain threshold (g) (±SD). Statistical analysis was performed using GraphPad and one-way ANOVA (n = 7). Model group vs. control group: #### p < 0.0001; Drug-treated groups vs. model group: **** p < 0.0001; ** p < 0.01; ns: not statistically significant.
FIG. 23 shows the mechanical pain threshold on Day 7 in the rat CCI model after administration of peptides at different doses in Example 9. The results are expressed as mechanical pain threshold (g) (±SD). Statistical analysis was performed using GraphPad and one-way ANOVA (n = 7). Model group vs. control group: #### p < 0.0001; Drug-treated groups vs. model group: **** p < 0.0001; ** p < 0.01; * p < 0.05; ns: not statistically significant.

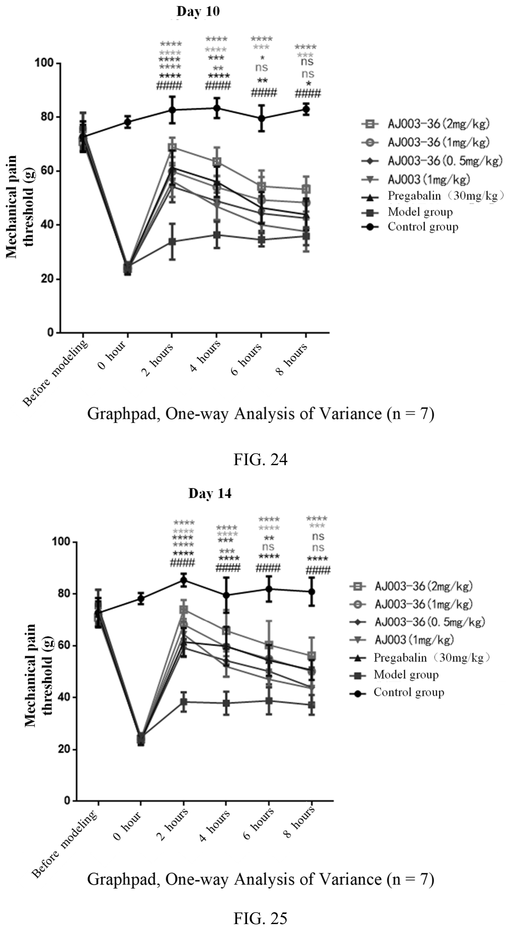

FIG. 24 shows the mechanical pain threshold on Day 10 in the rat CCI model after administration of peptides at different doses in Example 9. The results are expressed as mechanical pain threshold (g) (±SD). Statistical analysis was performed using GraphPad and one-way ANOVA (n = 7). Model group vs. control group: #### p < 0.0001; Drug-treated groups vs. model group: **** p < 0.0001; *** p < 0.001; ** p < 0.01; * p < 0.05; ns: not statistically significant.
FIG. 25 shows the mechanical pain threshold on Day 14 in the rat CCI model after administration of peptides at different doses in Example 9. The results are expressed as mechanical pain threshold (g) (±SD). Statistical analysis was performed using GraphPad and one-way ANOVA (n = 7). Model group vs. control group: #### p < 0.0001; Drug-treated groups vs. model group: **** p < 0.0001; *** p < 0.001; ** p < 0.01; ns: not statistically significant.

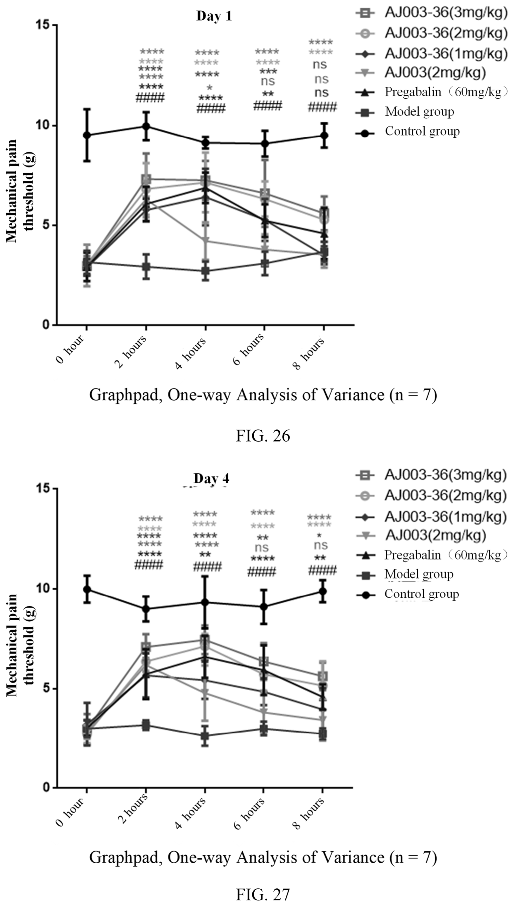

FIG. 26 shows the mechanical pain threshold on Day 1 in the mouse diabetic neuropathic pain model after administration of peptides at different doses in Example 10. The results are expressed as mechanical pain threshold (g) (±SD). Statistical analysis was performed using GraphPad and one-way ANOVA (n = 7). Model group vs. control group: #### p < 0.0001; Drug-treated groups vs. model group: **** p < 0.0001; *** p < 0.001; ** p < 0.01; * p < 0.05; ns: not statistically significant.
FIG. 27 shows the mechanical pain threshold on Day 4 in the mouse diabetic neuropathic pain model after administration of peptides at different doses in Example 10. The results are expressed as mechanical pain threshold (g) (±SD). Statistical analysis was performed using GraphPad and one-way ANOVA (n = 7). Model group vs. control group: #### p < 0.0001; Drug-treated groups vs. model group: **** p < 0.0001; *** p < 0.001; ** p < 0.01; * p < 0.05; ns: not statistically significant.

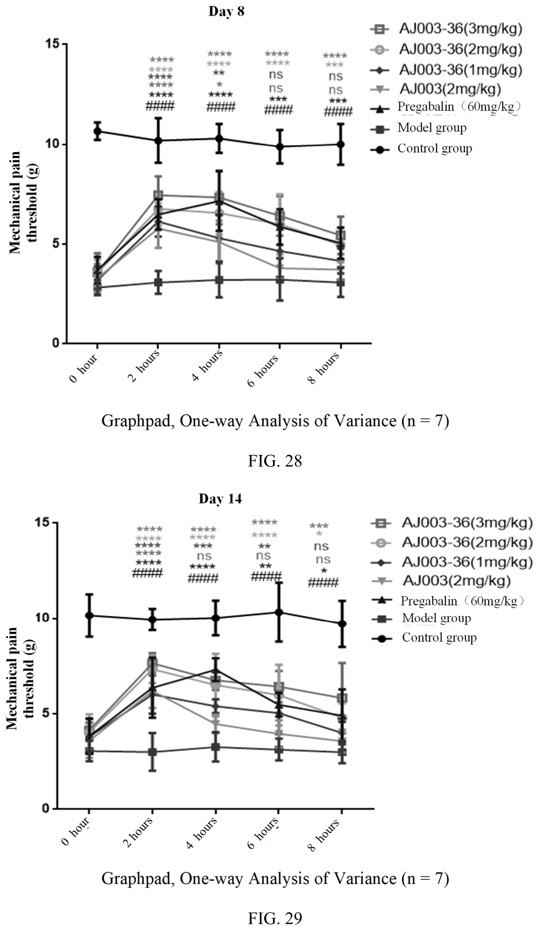

FIG. 28 shows the mechanical pain threshold on Day 8 in the mouse diabetic neuropathic pain model after administration of peptides at different doses in Example 10. The results are expressed as mechanical pain threshold (g) (±SD). Statistical analysis was performed using GraphPad and one-way ANOVA (n = 7). Model group vs. control group: #### p < 0.0001; Drug-treated groups vs. model group: **** p < 0.0001; *** p < 0.001; ** p < 0.01; * p < 0.05; ns: not statistically significant.
FIG. 29 shows the mechanical pain threshold on Day 14 in the mouse diabetic neuropathic pain model after administration of peptides at different doses in Example 10. The results are expressed as mechanical pain threshold (g) (±SD). Statistical analysis was performed using GraphPad and one-way ANOVA (n = 7). Model group vs. control group: #### p < 0.0001; Drug-treated groups vs. model group: **** p < 0.0001; *** p < 0.001; ** p < 0.01; * p < 0.05; ns: not statistically significant.

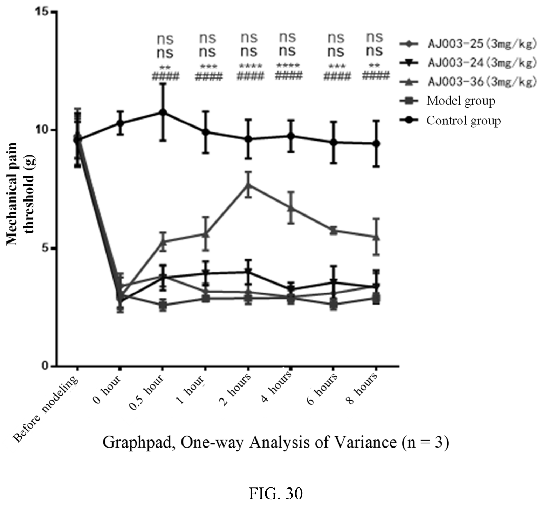

FIG. 30 illustrates the changes in mechanical pain threshold over time in a mouse oxaliplatin acute chemotherapy pain model after administration of different peptides in Example 8. The results are presented as mechanical pain threshold (g) (±SD). GraphPad, one-way ANOVA (n=3). Model group vs. control group: ####p < 0.0001; treatment group vs. model group: ****p < 0.0001; ***p < 0.001; **p < 0.01; ns: not statistically significant.

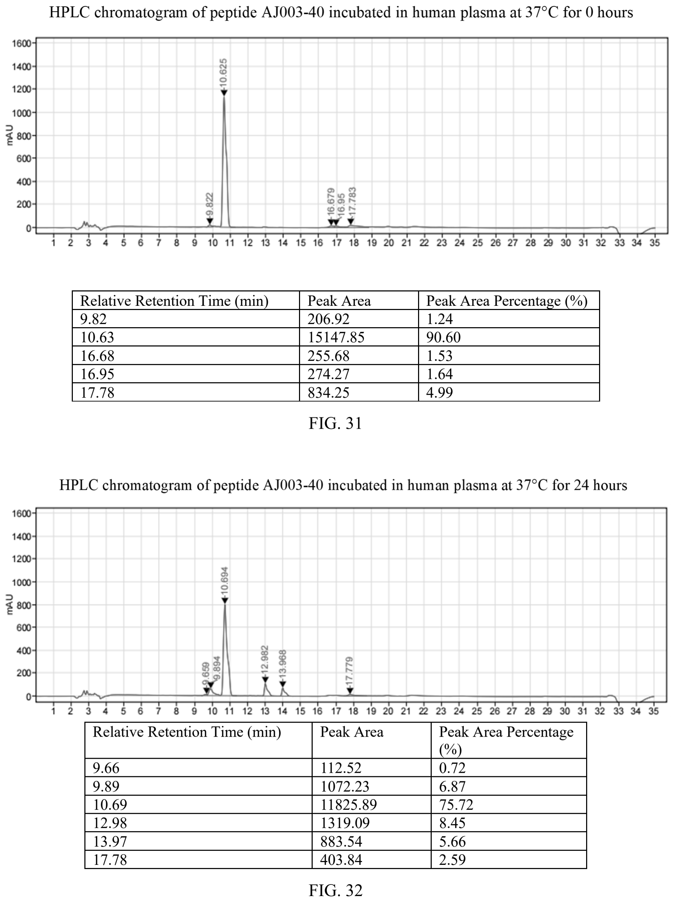

FIG. 31 shows the HPLC chromatogram of AJ003-40 incubated in human plasma at 37°C for 0 hours in Example 4.
FIG. 32 shows the HPLC chromatogram of AJ003-40 incubated in human plasma at 37°C for 24 hours in Example 4.

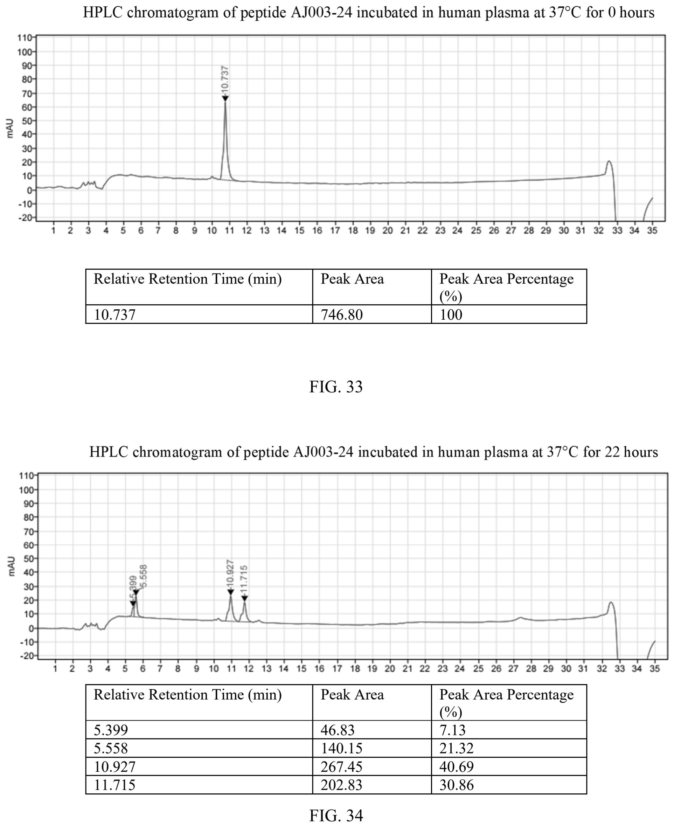

FIG. 33 shows the HPLC chromatogram of AJ003-24 incubated in human plasma at 37°C for 0 hours in Example 4.
FIG. 34 shows the HPLC chromatogram of AJ003-24 incubated in human plasma at 37°C for 22 hours in Example 4.

### DETAILED DESCRIPTION OF EMBODIMENTS

Unless otherwise stated, all numerical values representing content, concentration, ratio, weight, particle size, percentage, or technical effect in this specification and the claims should be understood as being modified by the terms "about" or "approximately." Therefore, unless indicated otherwise, the numerical parameters listed in this specification and the appended claims are approximate values.

Unless otherwise specified, the terminology used herein is intended to have the ordinary meaning as understood by those skilled in the art. Those skilled in the art can vary these parameters according to the desired properties and effects sought by this application. Each numerical parameter should be interpreted according to significant figures, conventional rounding methods, or as understood by those skilled in the art. In general, the nomenclature and the chemical, pharmaceutical, and biological experimental operations described herein are well-known to those of ordinary skill in the art and commonly employed in the relevant field. Unless otherwise defined, all technical and scientific terms used herein generally have the same meaning as commonly understood by a person skilled in the art. In cases where a term has multiple definitions, the definition provided in this section shall prevail unless indicated otherwise.

As used herein, the expression "A and/or B" includes three possibilities: (1) A; (2) B; and (3) both A and B. The expression "A, B, and/or C" includes seven possibilities: (1) A; (2) B; (3) C; (4) A and B; (5) A and C; (6) B and C; and (7) A, B, and C. Similar expressions should be interpreted analogously.

As used herein, the term "independently" means that multiple events occur without influencing each other. For example, "X and Y are independently selected from any one of a, b, c, d, e, f, g" means that X can be any one of a, b, c, d, e, f, g, and Y can also be any one of a, b, c, d, e, f, g; the selection of X and the selection of Y may be the same or different, both of which are independent of each other without mutual interference.

As used herein, alanine scanning (Cunningham and Wells, Science 244, 1081-1085, 1989) is employed to identify amino acids critical for the activity of the peptides of the present invention (e.g., binding affinity to acetylcholine receptors) so that these amino acids are not substituted. Alanine scanning introduces mutations at each residue of the molecule and evaluates the resulting molecule's biological activity to determine the amino acid residues essential for its activity.

As used herein, the terms "comprising" and "including" meaning that other elements are not excluded besides the listed elements.

As used herein, the term "identity" refers to the degree to which two (nucleotide or amino acid) sequences have identical residues at corresponding positions in an alignment and is generally expressed as a percentage. Preferably, identity is determined over the entire length of the compared sequences. Two copies having exactly the same sequence have 100% identity. Those skilled in the art will recognize that algorithms such as BLAST (Altschul et al., 1997, Nucleic Acids Res. 25:3389-3402), BLAST2 (Altschul et al., 1990, J. Mol. Biol. 215:403-410), Smith-Waterman (Smith et al., 1981, J. Mol. Biol. 147:195-197), and ClustalW can be used to determine sequence identity using standard parameters.

### Peptides or Derivatives Thereof

The present application provides a peptide or a derivative thereof comprising any one of the sequences selected from SEQ ID NOs: 1-34.

The present application further provides a peptide or a derivative thereof comprising any one of the sequences selected from SEQ ID NO: 1-34 having conservative substitutions. In some embodiments, any one of the sequences having conservative substitutions in SEQ ID NOs: 1-34 has 1, 2, 3, 4, or 5 conservative substitutions. In some embodiments, any one of the sequences having conservative substitutions in SEQ ID NOs: 1-34 has at least 65%, at least 70%, at least 75%, at least 80%, at least 84%, at least 85%, at least 90%, at least 92%, or at least 95% sequence identity with the corresponding sequence without conservative substitutions.

The present application provides a peptide or a derivative thereof comprising, in sequence from the N-terminus to the C-terminus, the following Formula I or Formula I with conservative substitutions:

Gly-Ser-Cys-Ser-X1-X2-X3-X4-(D-Cys)-X5-X6-X7-X8 Formula I

wherein the Cys at position 3 and the D-Cys at position 9 in Formula I form an intramolecular disulfide bond, D-Cys represents D-cysteine, and the "-" between amino acid residues represents a peptide bond. The C-terminus of the peptide or a derivative thereof is either a free carboxyl or an amide. X1 is Thr or D-Thr; X5 is selected from any one of Ala, Val, and D-Val; and X2-X4 and X6-X8 are independently any amino acid.

In some embodiments, X1 in Formula I is Thr or D-Thr. In some embodiments, X5 in Formula I is selected from any one of Ala, Val, and D-Val. In some embodiments, X2 in Formula I is selected from any one of Pro, D-Pro, and Hyp. In some embodiments, X3 in Formula I is selected from any one of Pro, D-Pro, and Hyp. In some embodiments, X4 in Formula I is selected from any one of Ser, D-Ser, Ala, and Aib. In some embodiments, X6 in Formula I is selected from any one of Ala, Leu, D-Leu, Ile, ABu, and Nle. In some embodiments, X7 in Formula I is selected from any one of Tyr, D-Tyr, Ala, Trp, Phe, and Bip. In some embodiments, X8 in Formula I is selected from any one of Ser, D-Ser, Ala, and Orn.

In some embodiments, Formula I with conservative substitutions has 1, 2, 3, 4, or 5 amino acid conservative substitutions. In an example of the present disclosure, Formula I with conservative substitutions has at least 65%, at least 70%, at least 75%, at least 80%, at least 84%, at least 85%, at least 90%, at least 92%, or at least 95% sequence identity with the corresponding Formula I without conservative substitutions.

In some embodiments, the peptide or a derivative thereof comprises any one sequence selected from the following sequences: SEQ ID NOs:1-5, SEQ ID NOs:10-16, and SEQ ID NOs:20-34, or any one of the sequences selected from SEQ ID NOs: 1-5, SEQ ID NOs: 10-16, and SEQ ID NOs: 20-34 with conservative substitutions. In some preferred embodiments, the peptide or a derivative thereof comprises SEQ ID NO:26 or SEQ ID NO:26 with conservative substitutions. In some preferred embodiments, the peptide or a derivative thereof comprises SEQ ID NO:28 or SEQ ID NO:28 with conservative substitutions. In some preferred embodiments, the peptide or a derivative thereof comprises SEQ ID NO:32 or SEQ ID NO:32 with conservative substitutions.

In some embodiments, the sequences with conservative substitutions have 1, 2, 3, 4, or 5 amino acid conservative substitutions. In some embodiments, sequences with conservative substitutions have at least 65%, at least 70%, at least 75%, at least 80%, at least 84%, at least 85%, at least 90%, at least 92%, or at least 95% sequence identity with the corresponding sequence without conservative substitutions.

In some embodiments, the derivative comprises one or more modifications selected from the following modifications: amino acid modifications, conservative amino acid substitutions, or substitution of hydrogen atoms in amino acid residues, as compared to the peptide.

As used herein, the term "peptide" refers to a compound in which amino acids are linked together by peptide bonds. Peptides consisting of three or more amino acid residues are referred to as polypeptides. Unless otherwise indicated or inconsistent with the context, the term "peptide" encompasses the peptide itself as well as its pharmaceutically acceptable salts, prodrugs, and metabolites. The peptides of the present application can be generated using techniques well-known in the art, including but not limited to solid-phase synthesis (e.g., Fmoc peptide synthesis) and solution-phase synthesis.

As used herein, the numbering of amino acid positions in a polypeptide or derivative is sequential from the N-terminus to the C-terminus. For example, the first amino acid from the N-terminus is the first amino acid, the second from the N-terminus is the second amino acid, the third is the third amino acid, and so on.

Unless otherwise defined, as commonly understood in the art, the left-hand end of an amino acid sequence or polypeptide sequence is the N-terminus (amino terminus), and the right-hand end is the C-terminus (carboxyl terminus). As used herein, the term "prodrug" refers to any molecule that can be converted in vivo under physiological conditions-for example, being transformed into the peptides or derivatives thereof described herein through reactions such as oxidation, reduction or hydrolysis under the action of enzymatic, gastric acidic conditions.

As used herein, the term "metabolite" refers to any molecule derived from the peptide or a derivative thereof described herein, in a cell or organism, preferably in a human.

As used herein with respect to peptides, the terms "amino acid" and "amino acid residue" have the same meaning and refer to: when amino acids are linked by chemical bonds, portions of the groups that participate in bond formation are lost, and the remaining portion of the amino acid is referred to as an amino acid residue.

As used herein, the names and abbreviations of amino acids correspond as follows:

| Name | Abbreviation | Single-letter Code |
|---|---|---|
| Glycine | Gly | G |
| Serine | Ser | S |
| Cysteine | Cys | C |
| Threonine | Thr | T |
| Proline | Pro | P |
| Hydroxyproline | Hyp | - |
| Alanine | Ala | A |
| Valine | Val | V |
| 2-Aminoisobutyric acid | Aib | - |
| Leucine | Leu | L |
| Isoleucine | Ile | I |
| 2-Aminobutyric acid | ABu | - |
| Norleucine | Nle | - |
| Tyrosine | Tyr | Y |
| Tryptophan | Trp | W |
| Phenylalanine | Phe | F |
| Biphenylalanine | Bip | - |
| Ornithine | Orn | - |
| Sarcosine | Sar | - |

Among natural amino acids, all except glycine comprise a chiral carbon atom. Unless otherwise specified, the optically active amino acids described herein are in the L-configuration. As customary, the peptide structures herein are written with the N-terminus on the left and the C-terminus on the right.

The term "amino acid" as used herein includes both natural amino acids and other "non-protein" α-amino acids/non-natural amino acids commonly used in peptide chemistry to prepare analogs of natural peptides.

Natural amino acids include glycine, alanine, valine, leucine, isoleucine, serine, methionine, threonine, phenylalanine, tyrosine, tryptophan, cysteine, proline, histidine, aspartic acid, asparagine, glutamic acid, glutamine, arginine, ornithine, and lysine. Examples of "non-protein" α-amino acids/non-natural amino acids include norleucine, norvaline, allo-isoleucine, homoarginine, thioproline, dehydroproline, hydroxyproline (Hyp), homoserine, cyclohexylglycine (Chg), α-amino-n-butyric acid (Aba), cyclohexylalanine (Cha), aminophenylbutyric acid (Pba), phenylalanine derivatives substituted on the phenyl ring with alkyl, alkoxy, halogen, or nitro groups, O-alkylated derivatives of serine, threonine, and tyrosine, S-alkylated cysteine, O-sulfated tyrosine, and D-isomers of natural amino acids.

As used herein, "peptide derivative" refers to a product obtained from the peptide through amino acid modifications, conservative amino acid substitutions, and/or substitution of hydrogen atoms in amino acid residues.

As used herein, types of "amino acid modifications" include, but are not limited to, N-terminal modifications, C-terminal modifications, and side-chain modifications. Modification methods include, but are not limited to, hydroxylation, carboxylation, alkylation, acylation, phosphorylation, sulfonation, amidation, aldehyde formation, alcohol formation, thiol-alkylation, esterification, glycosylation, and the like.

As used herein, a "conservative amino acid substitution" typically refers to a substitution of one amino acid residue with another amino acid having a similar chemical structure and/or similar chemical properties, which has minimal impact on the peptide's function, activity, or other biological properties. Such conservative substitutions are well known in the art. Examples include substituting an amino acid with another in the same group: (a) Small aliphatic, nonpolar or weakly polar residues: Ala, Ser, Thr, Pro, and Gly; (b) Negatively charged polar residues and their amides: Asp, Asn, Glu, and Gln; (c) Positively charged polar residues: His, Arg, and Lys; (d) Large aliphatic, nonpolar residues: Met, Leu, Ile, Val, and Cys; (e) Aromatic residues: Phe, Tyr, and Trp.

As used herein, substitution of hydrogen atoms in amino acid residues can be any conventional substituent known in the art, including but not limited to alkyl, alkenyl, alkynyl, aryl, alkoxy, carboxyl, aldehyde, carbonyl, hydroxyl, halogen, cyano, acyl, sulfonyl, amino, thiol, or nitro groups.

### Polynucleotides, Expression Vectors, and Host Cells

The present application provides a polynucleotide encoding the peptide or a derivative thereof described herein.

As used herein, a "polynucleotide" refers to a polymer of nucleotides (ribonucleotides or deoxyribonucleotides) of any length and refers to the primary structure of the molecule. This includes both double-stranded and single-stranded RNA, as well as double-stranded and single-stranded DNA. It also encompasses polynucleotides with modifications such as methylation and/or terminal modifications, as well as unmodified forms. The polynucleotides described herein are not necessarily obtained by physical methods and may be obtained by any method, including chemical synthesis, DNA replication, reverse transcription, or transcription.

The present application provides an expression vector capable of expressing the polynucleotide described herein.

The present application provides a host cell comprising the polynucleotide or the expression vector described herein.

As used herein, the term "host cell" refers to a cell that serves as a recipient for an expression vector or other introduced genetic fragments. Host cells include the originally transfected cells and their progeny. Host cells may be prokaryotic, yeast, or other eukaryotic cells.

### Pharmaceutical Compositions

The present application provides a pharmaceutical composition comprising the peptide or a derivative thereof disclosed herein, and a pharmaceutically acceptable carrier or salt.

The present application further provides a pharmaceutical composition comprising the peptide or a derivative thereof disclosed herein, a polynucleotide, an expression vector, or a host cell as disclosed herein, and a pharmaceutically acceptable carrier or salt.

As used herein, the term "pharmaceutical composition" refers to a mixture of the peptide disclosed herein with other chemical components such as carriers, stabilizers, diluents, dispersing agents, suspending agents, thickening agents, and/or excipients. Pharmaceutical compositions facilitate the administration of the peptide to a subject. Various administration routes for peptides are known in the art, including, but not limited to, subcutaneous, intramuscular, intravenous, intraperitoneal, intrathecal, oral, transdermal, pulmonary, ocular, and topical administration.

In the present application, the pharmaceutical composition may be formulated in a dosage form suitable for administration to a subject via the desired route. Such dosage forms include, but are not limited to, tablets, capsules, caplets, pills, lozenges, powders, syrups, elixirs, suspensions, solutions, emulsions, transdermal patches, suppositories, inhalants, creams, ointments, washes, pastes, sprays, lyophilized preparations, injectables, and gels.

As used herein, the term "pharmaceutically acceptable salt" includes acid addition salts and base addition salts. Suitable acid addition salts are formed from acids that produce non-toxic salts. Examples include, but are not limited to, acetate, hexanedioate, aspartate, benzoate, benzene sulfonate, bicarbonate/carbonate, bisulfate/sulfate, borate, camphorsulfonate, citrate, cyclohexylamine sulfonate, edetate, formate, fumarate, gluceptate, gluconate, glucuronate, hexafluorophosphate, 2-(4-hydroxybenzyl)benzoate, hydrochloride/chloride, hydrobromide/bromide, hydroiodide/iodide, 2-hydroxyethanesulfonate, lactate, malate, maleate, malonate, mesylate, methylsulfate, naphthalenesulfonate, nicotinate, nitrate, orotate, oxalate, palmitate, phosphate/hydrogen phosphate/dihydrogen phosphate, pyroglutamate, gluconate, stearate, salicylate, tannate, tartrate, toluenesulfonate, and trifluoroacetate. Suitable alkail addition salts are formed from alkail that produce non-toxic salts. Examples include, but are not limited to, aluminum, arginine, calcium, choline, diethanolamine, diethylamine, glycine, lysine, magnesium, meglumine, ethanolamine, potassium, sodium, tris(hydroxymethyl)aminomethane, and zinc salts. Acid and base half-salts may also be formed, such as bisulfates and hemicalcium salts. For a review of suitable salts, see Handbook of Pharmaceutical Salts: Properties, Selection and Use, Stahl and Wermuth (Wiley-VCH, 2002).

The term "pharmaceutically acceptable carrier" includes pharmaceutically acceptable materials, compositions, or carriers, such as liquid or solid fillers, diluents, excipients, solvents, or encapsulating materials, which are used to carry or deliver the peptide disclosed herein in a subject, such that it can perform its intended function. Each salt or carrier must be "acceptable" in the sense of being compatible with other components of the formulation and non-harmful to the subject. Examples of materials that can serve as pharmaceutically acceptable carriers include: sugars such as lactose, glucose, and sucrose; starches such as corn starch and potato starch; celluloses and derivatives such as sodium carboxymethylcellulose, ethylcellulose, and cellulose acetate; powdered tragacanth; malt; gelatin; talc; excipients such as cocoa butter and suppository waxes; oils such as peanut oil, cottonseed oil, safflower oil, sesame oil, olive oil, corn oil, and soybean oil; glycols such as propylene glycol; polyols such as glycerol, sorbitol, mannitol, and polyethylene glycol; esters such as ethyl oleate and ethyl laurate; agar; buffers such as magnesium hydroxide and aluminum hydroxide; alginates; pyrogen-free water; isotonic saline; Ringer's solution; ethanol; phosphate-buffered solutions; diluents; granulating agents; lubricants; binders; disintegrants; wetting agents; emulsifiers; coloring agents; release agents; coating agents; sweeteners; flavoring agents; fragrances; preservatives; antioxidants; plasticizers; gelling agents; thickeners; hardening agents; fixatives; suspending agents; surfactants; humectants; carriers; stabilizers; and other non-toxic compatible substances used in pharmaceutical formulations, or any combination thereof.

### Uses and Methods for Treating Diseases

The present application provides the use of the peptide or a derivative thereof, or the pharmaceutical composition disclosed herein, in the preparation of a medicament for treating pain.

The present application further provides the use of the peptide or a derivative thereof, the polynucleotide, the expression vector, the host cell, or the pharmaceutical composition disclosed herein, in the preparation of a medicament for treating pain.

In some embodiments, the medicament for treating pain is administered via subcutaneous, intramuscular, intravenous, intraperitoneal, intrathecal, oral, transdermal, pulmonary, ocular, or topical administration. In a preferred embodiment, the medicament for treating pain is administered subcutaneously.

In some embodiments, the pain is mediated via nicotinic acetylcholine receptors. In some embodiments, the pain includes neuropathic and inflammatory pain. In some embodiments, the pain includes chemotherapy-induced pain, diabetic peripheral neuropathic pain, sciatica, osteoarticular pain, postherpetic neuralgia, infection-induced pain, nutritional deficiencyinduced pain, inflammatory pain, chronic alcohol-induced pain, hypothyroidism-associated pain, autoimmune disease-related pain, Guillain-Barré syndrome pain, toxic pain, drug-induced pain, tumor-associated pain, genetic disorder-associated pain, and contusion pain.

The present application further provides the peptide or a derivative thereof, the polynucleotide, the expression vector, the host cell, or the pharmaceutical composition disclosed herein for use in treating pain.

In some embodiments, the pain is mediated via nicotinic acetylcholine receptors. In some embodiments, the pain includes neuropathic and inflammatory pain. In some embodiments, the pain includes chemotherapy-induced pain, diabetic peripheral neuropathic pain, sciatica, osteoarticular pain, postherpetic neuralgia, infection-induced pain, nutritional deficiencyinduced pain, inflammatory pain, chronic alcohol-induced pain, hypothyroidism-associated pain, autoimmune disease-related pain, Guillain-Barré syndrome pain, toxic pain, drug-induced pain, tumor-associated pain, genetic disorder-associated pain, and contusion pain.

In some embodiments, the peptide or a derivative thereof or the pharmaceutical composition is administered via subcutaneous, intramuscular, intravenous, intraperitoneal, intrathecal, oral, transdermal, pulmonary, ocular, or topical administration. In a preferred embodiment, the peptide or a derivative thereof or the pharmaceutical composition is administered subcutaneously.

The present application provides a method for treating pain, comprising administering to a subject in need thereof a therapeutically effective amount of the peptide or a derivative thereof, or the pharmaceutical composition disclosed herein.

The present application further provides a method for treating pain, comprising administering to a subject in need thereof a therapeutically effective amount of the peptide or a derivative thereof, a polynucleotide, an expression vector, a host cell, or the pharmaceutical composition disclosed herein.

In some embodiments, the pain is mediated via nicotinic acetylcholine receptors. In some embodiments, the pain includes neuropathic and inflammatory pain. In some embodiments, the pain includes chemotherapy-induced pain, diabetic peripheral neuropathic pain, sciatica, osteoarticular pain, postherpetic neuralgia, infection-induced pain, nutritional deficiencyinduced pain, inflammatory pain, chronic alcohol-induced pain, hypothyroidism-associated pain, autoimmune disease-related pain, Guillain-Barré syndrome pain, toxic pain, drug-induced pain, tumor-associated pain, genetic disorder-associated pain, and contusion pain.

In some embodiments, the peptide or a derivative thereof or the pharmaceutical composition is administered via subcutaneous, intramuscular, intravenous, intraperitoneal, intrathecal, oral, transdermal, pulmonary, ocular, or topical administration. In a preferred embodiment, the peptide or a derivative thereof or the pharmaceutical composition is administered subcutaneously.

As used herein, the term "subject" includes animals, such as vertebrates, preferably mammals, including dogs, cats, pigs, cows, sheep, horses, rodents (e.g., mice, rats, or guinea pigs), or primates (e.g., gorillas, chimpanzees, and humans).

As used herein, the term "treatment" refers to the alleviation or improvement of a disease or disorder (i.e., slowing or stopping the development of the disease or at least one clinical symptom), or the alleviation or improvement of at least one physical parameter or biomarker associated with the disease or disorder.

As used herein, the term "therapeutically effective amount" refers to an amount sufficient to provide a benefit or treat a disease in a subject relative to a corresponding subject who has not received such an amount, wherein such amount is sufficiently low to avoid serious adverse effects within reasonable medical judgment. The therapeutically effective amount of the peptide or a derivative thereof, polynucleotide, expression vector, host cell, or pharmaceutical composition disclosed herein may vary depending on the selected peptide or a derivative thereof, polynucleotide, expression vector, host cell, or pharmaceutical composition; the route of administration; the severity of the disease being treated; the age, body size, weight, and physical condition of the subject being treated; the medical history of the subject; the duration of treatment; the nature of any concurrent therapy; the desired therapeutic effect; and other factors, yet can be determined by those skilled in the art using routine methods.

The various embodiments described above for the peptide or a derivative thereof, polynucleotide, expression vector, and host cell disclosed herein are equally applicable to the pharmaceutical composition, uses, and methods disclosed herein (so long as they are not inherently contradictory), and any combination thereof forms part of the present disclosure.

### Examples

The following description illustrates exemplary examples of the present application with reference to the drawings, including various details of the examples to aid in understanding. It should be understood that these examples are merely illustrative and are in no way intended to limit the scope of the present application. The scope of the present application is defined solely by the claims. Those of ordinary skill in the art will recognize that various modifications and changes can be made to the examples described herein without departing from the scope of the present application. Likewise, for clarity and brevity, descriptions of well-known functions and structures are omitted in the following description.

Unless otherwise indicated, the reagents and instruments used in the following examples are conventional products commercially available. Unless otherwise stated, experiments are conducted under routine conditions or the conditions recommended by the manufacturer.

### Example 1: Synthesis of Conotoxin Peptides

Based on prior studies, a preliminary screening of peptide compounds was conducted through alanine scanning of the parent peptide AJ003, single-site incorporation of non-natural amino acids, and multi-site combinatorial modifications.

Synthesis of AJ003: Using Fmoc-Ser(tBu)-Wang resin (Tianjin Nankai Synthesis Technology Co., Ltd.) as the starting resin, a synthesis scale of 1 mmol was employed. The resin was added to a solid-phase peptide synthesizer (Nanjing Luanyu Chemical Glass Instrument Co., Ltd.), and swelled under nitrogen with dichloromethane for 30 minutes, followed by washing twice with DMF (dimethylformamide). A 20% piperidine/DMF solution was added twice for deprotection, each reaction lasting 5 minutes. After deprotection, the resin was washed 6 times with DMF, approximately 3 minutes per wash. Following the peptide sequence, standard solid-phase peptide synthesis principles were applied, coupling amino acids sequentially from the C-terminus to the N-terminus. The corresponding protected amino acids and coupling reagents (HOBt, DIC) were weighted and added and allowed to react for approximately 1 hour, followed by three washes with DMF. The coupling cycle (deprotection-wash-coupling-wash) was repeated, that is, 20% piperidine/DMF solution was added to the reaction vessel for deprotection , wash with DMF, and then add the protected amino acid and the corresponding coupling reagents for the coupling reaction. Using the coupling cycle method, protective amino acids are coupled to the N-terminus one by one, until all amino acids in the peptide were coupled. The N-terminal Fmoc group was removed using 20% piperidine/DMF, and the resin was alternately washed with dichloromethane and methanol. The peptide resin intermediate was obtained by vacuum drying. The peptide resin weight was recorded, and a cleavage solution was prepared at a ratio of 1 g resin: 10 mL cleavage solution. The cleavage solution composition was TFA:EDT:TIS:H₂O = 90:5:2.5:2.5. The resin was treated with the cleavage solution for approximately 3 hours. The filtrate was collected, and the crude peptide was precipitated using ice-cold methyl tert-butyl ether (MTBE) at an 8-fold volume relative to the filtrate. The mixture was stirred thoroughly and centrifuged to obtain crude peptide precipitate, which was washed with MTBE and vacuumdried overnight to yield linear crude peptide. The linear crude peptide was dissolved in water and oxidized using iodine to form the intramolecular disulfide bond. Oxidation proceeded for approximately 30 minutes, and the reaction progress was monitored by HPLC. Vitamin C was added to terminate the oxidation. The oxidized peptide solution was filtered and purified using reverse-phase high-performance liquid chromatography (RP-HPLC). Qualified fractions were combined and lyophilized to yield the final purified peptide.

Other peptides were synthesized following the same synthetic route as AJ003 using classic Fmoc solid-phase peptide synthesis to obtain linear crude peptides. For peptides with a C-terminal amidation, Rink amide MBHA resin was used. The crude peptide was oxidized using iodine to form disulfide-bonded peptide fractions, purified by RP-HPLC, and lyophilized to obtain the final peptide product. The peptide sequences involved in this example are shown in Table 1.

**Table 1 Summary of Peptide Sequences**

| SEQ ID NO | Peptide Code | Peptide Sequence |
|---|---|---|
| 1 | AJ003 | GSCSTPPS-(D-Cys)-VLYS (disulfide bond between positions 3 and 9) |
| 2 | AJ003-1 | GSCS-(D-Thr)-PPS-(D-Cys)-VLYS (3,9 disulfide bond) |
| 3 | AJ003-4 | GSCS-(D-Thr)-PPS-(D-Cys)-VLYS-NH₂ (3,9 disulfide bond) |
| 4 | AJ003-5 | GS-(D-Cys)-S-(D-Thr)-PPS-(D-Cys)-VLYS (3,9 disulfide bond) |
| 5 | AJ003-6 | G-(D-Ser)-(D-Cys)-(D-Ser)-(D-Thr)-(D-Pro)-(D-Pro)-(D- Ser)-(D-Cys)-(D-Val)-(D-Leu)-(D-Tyr)-(D-Ser) (3,9 disulfide bond) |
| 6 | AJ003-8 | Sar-SCS-(D-Thr)-PPS-(D-Cys)-VLYS (3,9 disulfide bond) |
| 7 | AJ003-12 | ASCS-(D-Thr)-PPS-(D-Cys)-VLYS (3,9 disulfide bond) |
| 8 | AJ003-13 | GACS-(D-Thr)-PPS-(D-Cys)-VLYS (3,9 disulfide bond) |
| 9 | AJ003-14 | GSCA-(D-Thr)-PPS-(D-Cys)-VLYS (3,9 disulfide bond) |
| 10 | AJ003-16 | GSCS-(D-Thr)-Hyp-PS-(D-Cys)-VLYS (3,9 disulfide bond) |
| 11 | AJ003-17 | GSCS-(D-Thr)-P-Hyp-S-(D-Cys)-VLYS (3,9 disulfide bond) |
| 12 | AJ003-18 | GSCS-(D-Thr)-PPA-(D-Cys)-VLYS (3,9 disulfide bond) |
| 13 | AJ003-19 | GSCS-(D-Thr)-PPS-(D-Cys)-ALYS (3,9 disulfide bond) |
| 14 | AJ003-20 | GSCS-(D-Thr)-PPS-(D-Cys)-VAYS (3,9 disulfide bond) |
| 15 | AJ003-21 | GSCS-(D-Thr)-PPS-(D-Cys)-VLAS (3,9 disulfide bond) |
| 16 | AJ003-22 | GSCS-(D-Thr)-PPS-(D-Cys)-VLYA (3,9 disulfide bond) |
| 17 | AJ003-24 | GSCS-(D-Thr)-PPG-(D-Cys)-VLYS (3,9 disulfide bond) |
| 18 | AJ003-25 | GSCS-(D-Thr)-PPS-(D-Cys)-Aib-LYS (3,9 disulfide bond) |
| 19 | AJ003-26 | GSCS-(D-Thr)-PPS-(D-Cys)-GLYS (3,9 disulfide bond) |
| 20 | AJ003-27 | GSCS-(D-Thr)-PPS-(D-Cys)-VIYS (3,9 disulfide bond) |
| 21 | AJ003-28 | GSCS-(D-Thr)-PPS-(D-Cys)-V-ABu-YS (3,9 disulfide bond) |
| 22 | AJ003-29 | GSCS-(D-Thr)-PPS-(D-Cys)-V-Nle-YS (3,9 disulfide bond) |
| 23 | AJ003-30 | GSCS-(D-Thr)-PPS-(D-Cys)-VLWS (3,9 disulfide bond) |
| 24 | AJ003-32 | GSCS-(D-Thr)-PPS-(D-Cys)-VL-Bip-S (3,9 disulfide bond) |
| 25 | AJ003-33 | GSCS-(D-Thr)-PPS-(D-Cys)-VLY-Orn-NH₂ (3,9 disulfide bond) |
| 26 | AJ003-34 | GSCS-(D-Thr)-Hyp-Hyp-S-(D-Cys)-V-Nle-Bip-Orn-NH₂ (3,9 disulfide bond) |
| 27 | AJ003-35 | GSCS-(D-Thr)-Hyp-Hyp-A-(D-Cys)-V-Nle-Bip-Orn-NH₂ (3,9 disulfide bond) |
| 28 | AJ003-36 | GSCS-(D-Thr)-Hyp-Hyp-Aib-(D-Cys)-V-Nle-Bip-Orn-NH₂ (3,9 disulfide bond) |
| 29 | AJ003-37 | GSCS-(D-Thr)-Hyp-Hyp-S-(D-Cys)-V-Nle-F-Orn-NH₂ (3,9 disulfide bond) |
| 30 | AJ003-38 | GSCS-(D-Thr)-Hyp-Hyp-A-(D-Cys)-V-Nle-F-Orn-NH₂ (3,9 disulfide bond) |
| 31 | AJ003-39 | GSCS-(D-Thr)-Hyp-Hyp-Aib-(D-Cys)-V-Nle-F-Orn-NH₂ (3,9 disulfide bond) |
| 32 | AJ003-40 | GSCS-(D-Thr)-Hyp-Hyp-S-(D-Cys)-V-Nle-W-Orn-NH₂ (3,9 disulfide bond) |
| 33 | AJ003-41 | GSCS-(D-Thr)-Hyp-Hyp-A-(D-Cys)-V-Nle-W-Orn-NH₂ (3,9 disulfide bond) |
| 34 | AJ003-42 | GSCS-(D-Thr)-Hyp-Hyp-Aib-(D-Cys)-V-Nle-W-Orn-NH₂ (3,9 disulfide bond) |

| | | |
|---|---|---|
| *D-Thr: D-threonine; 3,9 disulfide bond: disulfide bond formed between the 3rd and 9th amino acid residues of the sequence. | | |

### Example 2: Determination of Peptide-Target Protein Affinity by BLI (Biolayer Interferometry)

BLI is a label-free technology that converts optical interference signals occurring on the surface of BLI biosensors into real-time response signals. By monitoring the molecular binding process in real time, the system determines association rate constants (ka or kon), dissociation rate constants (kd or koff), and initial binding rates, and calculates affinity (K_{D}) by fitting analysis. Here are the specific procedures: NTA sensors (Sartorius) were placed in a pre-wetting tray, which was then positioned in a black 96-well plate with 200 µL PBS per well. The NTA sensors were pre-wetted for at least 15 minutes. The target protein α9 nAChR was diluted in PBS to 5 µg/mL, with 200 µL added per well. Test peptides were dissolved in PBS and serially diluted threefold, with concentrations from high to low as 1000 nM, 333.3 nM, 111.1 nM, 37.04 nM, 12.35 nM, 4.115 nM, and 1.372 nM, with an additional well with PBS only as a zeroconcentration control. After equilibrating the instrument baseline, the NTA sensor was used to immobilize the target protein α9 nAChR. Once immobilization was complete, the baseline was re-equilibrated, followed by a 5-minute association and a 5-minute dissociation phase. Affinity measurements of the test peptides were conducted sequentially using the above procedure. The procedure for measuring peptide affinity to α7 nAChR was similar, except that the α7 nAChR concentration was 20 µg/mL. Tables 2 and 3 show the BLI-determined affinities of alanine-scan peptide analogs with target proteins, which identified sites on the parent peptide suitable for modification. Subsequently, single-site non-natural amino acid substituted peptides were evaluated by BLI for target protein affinity. Finally, multi-site combinatorial modified peptides were measured for affinity by BLI, leading to the selection of high-affinity peptides for further screen and drug development potential.

**Table 2 Test Results for Affinity of Modified Peptides to α9 nAChR**

| Subtype of Target Protein | Peptide Name | SEQ ID NO | KD (M) | R² |
|---|---|---|---|---|
| α9 nAChR | AJ003 | 1 | 4.480E-09 | 0.9668 |
| | AJ003-1 | 2 | 8.597E-08 | 0.9582 |
| | AJ003-12 | 7 | 3.164E-05 | 0.907 |
| | AJ003-13 | 8 | 6.692E-06 | 0.8692 |
| | AJ003-14 | 9 | 1.394E-05 | 0.7426 |
| | AJ003-16 | 10 | 3.532E-08 | 0.8284 |
| | AJ003-17 | 11 | 1.097E-07 | 0.9197 |
| | AJ003-18 | 12 | 4.264E-08 | 0.7964 |
| | AJ003-19 | 13 | 3.173E-07 | 0.8726 |
| | AJ003-20 | 14 | 1.632E-08 | 0.9347 |
| | AJ003-21 | 15 | 5.142E-07 | 0.5396 |
| | AJ003-22 | 16 | 1.634E-07 | 0.9068 |

**Table 3 Test Results for Affinity of Modified Peptides to α7 nAChR**

| Subtype of Target Protein | Peptide Name | SEQ ID NO | KD (M) | R² |
|---|---|---|---|---|
| α7 nAChR | AJ003 | 1 | 2.232E-07 | 0.8646 |
| | AJ003-1 | 2 | 2.202E-08 | 0.8363 |
| | AJ003-4 | 3 | 3.387E-07 | 0.9225 |
| | AJ003-5 | 4 | 4.004E-07 | 0.9354 |
| | AJ003-6 | 5 | 5.322E-07 | 0.9223 |
| | AJ003-8 | 6 | 6.420E-06 | 0.826 |
| | AJ003-24 | 17 | 1.398E-05 | 0.9479 |
| | AJ003-25 | 18 | 4.665E-06 | 0.9572 |
| | AJ003-26 | 19 | 6.829E-06 | 0.9285 |
| | AJ003-27 | 20 | 3.232E-07 | 0.8957 |
| | AJ003-28 | 21 | 6.898E-07 | 0.7547 |
| | AJ003-29 | 22 | 6.738E-07 | 0.8916 |
| | AJ003-30 | 23 | 2.780E-08 | 0.8435 |
| | AJ003-32 | 24 | 3.751E-07 | 0.9059 |
| | AJ003-33 | 25 | 3.998E-07 | 0.8112 |
| | AJ003-34 | 26 | 3.597E-07 | 0.9317 |
| | AJ003-35 | 27 | 1.425E-07 | 0.88 |
| | AJ003-36 | 28 | 4.080E-08 | 0.9165 |
| | AJ003-37 | 29 | 6.867E-08 | 0.8223 |
| | AJ003-38 | 30 | 1.867E-07 | 0.8901 |
| | AJ003-39 | 31 | 4.099E-07 | 0.8756 |
| | AJ003-40 | 32 | 4.645E-08 | 0.8453 |
| | AJ003-41 | 33 | 1.095E-07 | 0.855 |
| | AJ003-42 | 34 | 2.116E-08 | 0.8103 |

### Example 3: In Vitro Plasma Stability and Predicted Half-Life of Peptides

Peptides generally exhibit short half-lives and are easily degraded in vivo by various enzymes and blood matrix components. The modified peptides in this study were designed with non-natural amino acid substitutions at certain non-essential positions to enhance peptide stability and improve druggability. Peptides were dissolved in deionized water to 2 mg/mL, then diluted with acetonitrile to 1 mg/mL and filtered through an organic membrane. 200 µL of the filtrate was transferred to an LC vial as the peptide control. 300 µL of human plasma (comprising EDTA as anticoagulant, Nanjing Senbeijia Biotechnology Co., Ltd., Lot No. SBJ-P-HU011-100 mL) was mixed with 300 µL acetonitrile, centrifuged at 4°C and 12,000 rpm for 5 minutes. The supernatant was filtered through an organic membrane, and 200 µL of the filtrate was transferred to an LC vial as the plasma blank control. For peptide samples, 10 mg of peptide was weighed mixed with 5 mL of human plasma and vortexed thoroughly. 300 µL of peptide-containing plasma was mixed with 300 µL acetonitrile, centrifuged at 4°C and 12,000 rpm for 5 minutes, and the supernatant was filtered through an organic membrane. 200 µL of the filtrate was transferred to an LC vial as the 0 min sample. Peptide-containing plasma was incubated in a 37°C water bath, and samples at other time points were collected using the same procedure as the 0 min sample. For peptides AJ003 and AJ003-1, the sampling time points were 0 min, 5 min, 10 min, 20 min, 30 min, 1 h, 2 h, 4 h, 6 h, 10 h, 12 h, and 24 h. For modified peptides AJ003-34, AJ003-35, AJ003-36, AJ003-37, AJ003-38, AJ003-39, AJ003-40, AJ003-41, and AJ003-42, the time points were 0 min, 10 min, 30 min, 1 h, 4 h, 6 h, 10 h, 12 h, and 24 h. All peptide sample series were analyzed by HPLC, recording the changes in main peak areas over plasma incubation time. Predicted in vitro half-lives were calculated using the pharmacokinetic software Winolin. Due to the presence of tautomers, AJ003 peak areas were difficult to integrate accurately over time. AJ003-1 is an analog of AJ003 in which the threonine at position 5 is substituted with D-threonine; its sequence differs from AJ003 by only one amino acid configuration. As shown in FIGS. 1-10, the in vitro plasma half-life of AJ003-1 was approximately 10.8 h. The in vitro plasma half-lives of modified peptides AJ003-34, AJ003-35, AJ003-36, AJ003-37, AJ003-38, AJ003-39, AJ003-40, AJ003-41, and AJ003-42 were 61.9 h, 45.3 h, 65.3 h, 71.0 h, 26.7 h, 43.0 h, 84.0 h, 30.0 h, and 61.4 h, respectively. Overall, through non-natural amino acid substitutions, the modified peptides in this project exhibited significantly improved in vitro plasma stability compared with AJ003-1.

### Example 4: Plasma Stability Experiments for AJ003-40 and AJ003-24

Peptides AJ003-40 and AJ003-24 were separately weighed and mixed with human plasma to prepare solutions at 2 mg/mL. 300 µL of peptide-containing plasma was mixed with 300 µL acetonitrile, centrifuged at 4°C and 12,000 rpm for 5 minutes, and the supernatant was filtered through an organic membrane. 200 µL of the filtrate was transferred to an LC vial as the 0 h sample. Peptide-containing plasma was incubated in a 37°C water bath. For AJ003-40, 300 µL of plasma was sampled at 24 h, mixed with 300 µL acetonitrile, centrifuged, filtered, and 200 µL of the filtrate was transferred to an LC vial as the 24 h sample. For AJ003-24, 300 µL of plasma was sampled at 22 h, processed similarly, and 200 µL of the filtrate was transferred to an LC vial as the 22 h sample. HPLC analysis of 0 h and 24 h (or 22 h) samples, as shown in Figures 31 and 32, for AJ003-40, the main peak area decreased from 15149.38 at 0 h to 11829.51 at 24 h, corresponding to a 21.9% degradation. For AJ003-24, as shown in Figures 33 and 34, the main peak area decreased from 746.80 at 0 h to 267.45 at 22 h, corresponding to a 64.2% degradation. Compared with AJ003-40, AJ003-24 degraded more rapidly, indicating that AJ003-24 has lower plasma stability than AJ003-40.

### Example 5: In Vivo Analgesic Activity of Selected Modified Peptides in the Paclitaxel Chemotherapy Pain Model at the Same Dose

Male SPF (Specific Pathogen Free) SD rats weighing 180-200 g (Shanghai Institute of Planned Parenthood Research, Experimental Animal Department) were used. On the day of model induction, rats were weighed, and baseline mechanical pain thresholds were measured. Animals with large differences in body weight or pain threshold were excluded, and the remaining rats were subjected to the experimental protocol. Paclitaxel powder was dissolved in olive oil to 1 mg/mL. Each rat received an intraperitoneal injection of 2 mg/kg per dose. Four injections were administered on days 1, 3, 5, and 7. Rats in the negative control or vehicle control group received injections of the solvent used to dissolve paclitaxel. According to literature reports, this model exhibits a significant reduction in mechanical pain threshold approximately 15 days after model induction, indicating the successful establishment of a stable chronic paclitaxel-induced chemotherapy pain model in rats. Drug treatment was carried out according to the experimental dosing schedule shown in Table 4. The blank group in Table 4 represents rats without pain modeling, and the model group represents rats with pain modeling but no drug treatment. On days 1, 3, 5, and 12, drugs were administered at fixed times. On days 1, 3, and 5, mechanical pain thresholds of the right hind paw at the corresponding time were measured at 1 h, 2.5 h, 4 h, and 5.5 h after dosing using a blunt probe. On day 12, mechanical pain thresholds were measured at 4 h and 7 h after dosing. As shown in FIGs. 11-14, after four rounds of treatment, the parent peptide AJ003 demonstrated significant analgesic activity within 4 h compared with the model group, but no significant analgesic effect was observed beyond 4 h. Among the five candidate modified peptides (AJ003-34, AJ003-35, AJ003-36, AJ003-39, AJ003-40), AJ003-34, AJ003-36, and AJ003-40 were identified as the top three analgesic candidates. Analysis of day 12 at 7 h showed that AJ003-36 and AJ003-40 exhibited prolonged analgesic activity lasting up to 7 h. Compared with the positive control drug pregabalin, these peptides demonstrated slightly superior analgesic activity at certain time points, and overall were comparable in analgesic efficacy to pregabalin. AJ003-36 and AJ003-40 were thus identified as candidate molecules for further study. Paclitaxel is a widely used small-molecule chemotherapy drug that, while effective against tumors, can induce neuropathic pain in many cancer patients due to neurotoxicity. The chronic chemotherapy pain model induced in male SD rats via multiple low-dose intraperitoneal injections of paclitaxel simulates clinical chemotherapy-induced neuropathic pain. Using this model, the analgesic effects of five modified peptides, the parent peptide AJ003, and the positive control pregabalin were evaluated. Results indicate that mechanical pain thresholds of the right hind paw after each administration can be used to preliminarily identify AJ003-36 and AJ003-40 as candidate molecules for further development.

**Table 4 Dosing Scheme for Screening Analgesic Activity of Selected Modified Peptides at the Same Dose in the Paclitaxel Chemotherapy Pain Model**

| Group | Drug | Administration Route | Dose | Number of Animals | Dosing Frequency |
|---|---|---|---|---|---|
| G1 (Blank/Control) | Saline | Subcutaneous injection | 0 mg/kg (0.4 mL/200 g) | 8 | Once daily for 4 days |
| G2 (Model) | Saline | Subcutaneous injection | 0 mg/kg (0.4 mL/200 g) | 8 | Once daily for 4 days |
| G3 (Positive Control) | Pregabalin | Oral gavage | 30 mg/kg | 8 | Once daily for 4 days |
| G4 (AJ003) | AJ003 | Subcutaneous injection | 1 mg/kg | 8 | Once daily for 4 days |
| G5 (AJ003-34) | AJ003-34 | Subcutaneous injection | 1 mg/kg | 8 | Once daily for 4 days |
| G6 (AJ003-35) | AJ003-35 | Subcutaneous injection | 1 mg/kg | 8 | Once daily for 4 days |
| G7 (AJ003-36) | AJ003-36 | Subcutaneous injection | 1 mg/kg | 8 | Once daily for 4 days |
| G8 (AJ003-39) | AJ003-39 | Subcutaneous injection | 1 mg/kg | 8 | Once daily for 4 days |
| G9 (AJ003-40) | AJ003-40 | Subcutaneous injection | 1 mg/kg | 8 | Once daily for 4 days |

### Example 6: In Vivo Analgesic Activity of Modified Peptides at Different Doses in the Paclitaxel Chemotherapy Pain Model

Male SPF (Specific Pathogen Free) SD rats weighing 180-200 g (Shanghai Institute of Planned Parenthood Research, Experimental Animal Department) were purchased and allowed to acclimate for approximately one week. On the day of model induction, rats were weighed, and baseline mechanical pain thresholds were measured. Animals with large differences in body weight or pain threshold were excluded, and the remaining rats were subjected to the experimental protocol. Paclitaxel powder was dissolved in olive oil to 1 mg/mL. Each rat received an intraperitoneal injection of 2 mg/kg per dose. Four injections were administered on days 1, 3, 5, and 7. Rats in the negative control or vehicle control group received injections of the solvent used to dissolve paclitaxel. According to literature reports, this model exhibits a significant reduction in mechanical pain threshold approximately 15 days after model induction, indicating the successful establishment of a stable chronic paclitaxel-induced chemotherapy pain model in rats. Drug treatment was carried out according to the dosing schedule shown in Table 5. Starting from day 15 after model induction, drugs were administered daily for consecutive days. On days 1, 4, 7, 12, and 14 of treatment, mechanical pain thresholds of the right hind paw at the corresponding time were measured at 2 h, 4 h, 6 h, and 8 h after dosing using a blunt probe. Results are shown in FIGs. 15-19. Analysis of five rounds of treatment showed that the parent peptide AJ003 exhibited significant analgesic activity within 4 h compared with the model group, but no significant effect was observed beyond 4 h. The two candidate modified peptides, AJ003-36 and AJ003-40, demonstrated analgesic activity at a low dose of 0.5 mg/kg comparable to that of AJ003 at 1 mg/kg, and in some time points on certain treatment days, their effect exceeded that of the parent peptide AJ003. At a high dose of 1.5 mg/kg, AJ003-36 and AJ003-40 exhibited analgesic activity slightly superior to the positive control pregabalin, and their analgesic effect remained significant even 8 h after dosing compared with the model group.

**Table 5 Dosing Scheme for Screening Analgesic Activity of Selected Modified Peptides at Different Doses in the Paclitaxel Chemotherapy Pain Model**

| Group | Drug | Administration Route | Dose | Number of Animals | Dosing Frequency |
|---|---|---|---|---|---|
| G1 (Blank/Control) | Saline | Subcutaneous injection | 0 mg/kg (0.4 mL/200 g) | 8 | Once daily for 14 days |
| G2 (Model) | Saline | Subcutaneous injection | 0 mg/kg (0.4 mL/200 g) | 8 | Once daily for 14 days |
| G3 (Positive Control) | Pregabalin | Oral gavage | 30 mg/kg | 8 | Once daily for 14 days |
| G4 (AJ003) | AJ003 | Subcutaneous injection | 1 mg/kg | 8 | Once daily for 14 days |
| G5 (AJ003-36 Low Dose) | AJ003-36 | Subcutaneous injection | 0.5 mg/kg | 8 | Once daily for 14 days |
| G6 (AJ003-36 High Dose) | AJ003-36 | Subcutaneous injection | 2 mg/kg | 8 | Once daily for 14 days |
| G7 (AJ003-40 Low Dose) | AJ003-40 | Subcutaneous injection | 0.5 mg/kg | 8 | Once daily for 14 days |
| G8 (AJ003-40 High Dose) | AJ003-40 | Subcutaneous injection | 2 mg/kg | 8 | Once daily for 14 days |

### Example 7: In Vivo Analgesic Activity of Candidate Modified Peptides at Different Doses in the Oxaliplatin Chemotherapy Pain Model

Oxaliplatin was dissolved in 5% glucose solution to a concentration of 1 mg/mL. For model induction, each male C57 mouse (Hangzhou Ziyuan Experimental Animal Technology Co., Ltd.) received a single intraperitoneal injection of oxaliplatin at 10 mg/kg. Mice in the negative control or blank control group received 5% glucose solution. Approximately one week after model induction, mechanical pain thresholds of the mice were measured three times per mouse, and the average value was calculated. Mice were then randomly assigned to groups based on their pain thresholds. Drug administration was carried out according to the dosing schedule shown in Table 6, with a single dose per mouse. Mechanical pain thresholds were measured at 0.5, 1, 2, 4, 6, and 8 h post-dose. As shown in FIG. 20, at 0.5, 1, 2, and 4 h post-dose, the positive control pregabalin, the parent peptide AJ003, and the low, medium, and high dose groups of the candidate modified peptide AJ003-36 all exhibited significant analgesic activity compared with the model group. At 6 h post-dose, the parent peptide AJ003 showed no significant effect compared with the model group, whereas pregabalin and all three doses of AJ003-36 still demonstrated significant analgesic activity. At 8 h post-dose, the parent peptide AJ003 and the low-dose AJ003-36 group showed no significant effect compared with the model group, while pregabalin and the medium- and high-dose groups of AJ003-36 continued to show significant analgesic activity.

**Table 6 Dosing Scheme for Oxaliplatin Chemotherapy Pain Model**

| Group | Drug | Administration Route | Dose | Number of Animals | Dosing Frequency |
|---|---|---|---|---|---|
| G1 (Blank/Control) | Saline | Subcutaneous injection | 0 mg/kg (10 mL/kg) | 8 | Single dose |
| G2 (Model) | Saline | Subcutaneous injection | 0 mg/kg (10 mL/kg) | 8 | Single dose |
| G3 (Positive Control) | Pregabalin | Oral gavage | 60 mg/kg (6 mL/kg) | 8 | Single dose |
| G4 (AJ003) | AJ003 | Subcutaneous injection | 2 mg/kg (10 mL/kg) | 8 | Single dose |
| G5 (AJ003-36 Low Dose) | AJ003-36 | Subcutaneous injection | 1 mg/kg (10 mL/kg) | 8 | Single dose |
| G6 (AJ003-36 Medium Dose) | AJ003-36 | Subcutaneous injection | 2 mg/kg (10 mL/kg) | 8 | Single dose |
| G7 (AJ003-36 High Dose) | AJ003-36 | Subcutaneous injection | 3 mg/kg (10 mL/kg) | 8 | Single dose |

### Example 8: In Vivo Analgesic Activity of Multiple Peptides in the Oxaliplatin Chemotherapy Pain Model

Oxaliplatin was dissolved in 5% glucose solution to a concentration of 1 mg/mL. For model induction, each male C57 mouse (Shanghai Bikai Keyi Biotechnology Co., Ltd.) received a single intraperitoneal injection of oxaliplatin at 10 mg/kg. Mice in the negative control or blank control group received 5% glucose solution. Approximately one week after model induction, mechanical pain thresholds of the mice were measured three times per mouse, and the average value was calculated. Mice were then randomly assigned to groups based on their pain thresholds. Drug administration was carried out according to the dosing schedule shown in Table 7, with a single dose per mouse. Mechanical pain thresholds were measured at 0.5, 1, 2, 4, 6, and 8 h post-dose. As shown in FIG. 30, beginning at 0.5 h post-dose, the AJ003-36 treated group showed a significant decrease in mechanical pain threshold compared with the model group, indicating effective analgesic activity against oxaliplatin-induced chemotherapy pain. In contrast, the AJ003-24 and AJ003-25 treated groups did not show a significant decrease in mechanical pain threshold, suggesting that these peptides were ineffective in alleviating chemotherapy-induced pain in mice.

**Table 7 Dosing Scheme for Oxaliplatin Chemotherapy Pain Model in Mice**

| | | | | | |
|---|---|---|---|---|---|
| Group | Drug | Administration Route | Dose | Number of Animals | Dosing Frequency |
| G1 (Blank/Control) | Saline | Subcutaneous injection | 0 mg/kg (10 mL/kg) | 3 | Single dose |
| G2 (Model) | Saline | Subcutaneous injection | 0 mg/kg (10 mL/kg) | 3 | Single dose |
| G3 (AJ003-36) | AJ003-36 | Subcutaneous injection | 3 mg/kg (10 mL/kg) | 3 | Single dose |
| G4 (AJ003-24) | AJ003-24 | Subcutaneous injection | 3 mg/kg (10 mL/kg) | 3 | Single dose |
| G5 (AJ003-25) | AJ003-25 | Subcutaneous injection | 3 mg/kg (10 mL/kg) | 3 | Single dose |

### Example 9: Evaluation of In Vivo Analgesic Activity of Candidate Modified Peptides at Different Doses in the Rat CCI Model

Neuropathic pain has many types, and animal models can simulate clinical neuropathic pain. Common SD rat sciatic nerve neuropathic pain models include CCI (Chronic Constriction Injury), SNI (Spared Nerve Injury), SNL (Spinal Nerve Ligation), and CCD (using stainless steel wire through the intervertebral foramen to fix and compress the dorsal root ganglion). Among them, the CCI model is widely used to study neuropathic pain, providing a basis for further research on mechanisms and strategies for nerve injury repair (Wen Xiaojun, Medical Information, Jan. 2018, Vol. 31, No.1). In this example, the CCI model was employed.

Except for the control group, the remaining SD rats were anesthetized via intraperitoneal injection of 10% chloral hydrate saline solution at a dose of 0.3 mL per 100 g body weight, and surgical modeling was performed. The right hind limb skin was carefully incised, muscles were separated, and the sciatic nerve was ligated three times using 4-0 surgical silk, spaced 1 mm apart. Muscles and skin were sutured, and antibiotics were applied to the wound to prevent infection. According to literature reports, 7-10 days after surgery, animals exhibit a significant decrease in pain threshold, which can persist for approximately one month. Approximately 10 days post-surgery, plantar pain thresholds were measured three times for each rat, and the average value was calculated. Rats were randomly assigned to groups based on pain thresholds. Drug administration was performed according to Table 8, and plantar pain thresholds were measured at 2 h, 4 h, 6 h, and 8 h after administration.

Day 1 of dosing (as shown in FIG. 21): At 2 h and 4 h, pregabalin, the parent peptide AJ003, and candidate modified peptide AJ003-36 at low, medium, and high doses all showed significant analgesic activity compared with the model group. At 6 h, AJ003 showed no significant difference compared with the model group, while pregabalin and AJ003-36 at all doses still showed significant analgesic activity. At 8 h, AJ003 and AJ003-36 low-dose groups were not significantly different from the model group, while pregabalin and AJ003-36 medium/high dose groups still showed significant analgesic activity.

Day 4 of dosing (as shown in FIG. 22): At 2 h and 4 h, pregabalin, AJ003, and AJ003-36 at low, medium, and high doses all showed significant analgesic activity. At 6 h and 8 h, AJ003 and low-dose AJ003-36 were no longer significant compared with the model group; pregabalin and AJ003-36 medium/high dose groups remained significant.

Day 7 of dosing (as shown in FIG. 23): At 2 h and 4 h, pregabalin, AJ003, and AJ003-36 at low, medium, and high doses all showed significant analgesic activity. At 6 h, AJ003 showed no significant effect compared with the model group, while pregabalin and all AJ003-36 doses were still significant. At 8 h, AJ003 and AJ003-36 low-dose groups were not significant, while pregabalin and AJ003-36 medium/high dose groups remained significant.

Day 10 of dosing (as shown in FIG. 24): At 2 h and 4 h, pregabalin, AJ003, and AJ003-36 at low, medium, and high doses all showed significant analgesic activity. At 6 h, AJ003 showed no significant effect, while pregabalin and all AJ003-36 doses remained significant. At 8 h, AJ003 and low-dose AJ003-36 were not significant, whereas pregabalin and AJ003-36 medium/high dose groups remained significant.

Day 14 of dosing (as shown in FIG. 25): At 2 h and 4 h, pregabalin, AJ003, and AJ003-36 at low, medium, and high doses all showed significant analgesic activity. At 6 h, AJ003 showed no significant effect, while pregabalin and all AJ003-36 doses remained significant. At 8 h, AJ003 and low-dose AJ003-36 were not significant, whereas pregabalin and AJ003-36 medium/high dose groups remained significant.

**Table 8 Dosing Scheme for Rat CCI Model**

| Group | Drug | Administration Route | Dose | Number of Animals | Dosing Frequency |
|---|---|---|---|---|---|
| G1 (Blank/Control) | Saline | Subcutaneous injection | 0 mg/kg (1 mL/kg) | 7 | Once daily for 14 days |
| G2 (Model) | Saline | Subcutaneous injection | 0 mg/kg (1 mL/kg) | 7 | Once daily for 14 days |
| G3 (Positive Drug) | Pregabalin | Oral | 30 mg/kg (1 mL/kg) | 7 | Once daily for 14 days |
| G4 (AJ003) | AJ003 | Subcutaneous injection | 1 mg/kg (1 mL/kg) | 7 | Once daily for 14 days |
| G5 (AJ003-36 Low Dose) | AJ003-36 | Subcutaneous injection | 0.5 mg/kg (1 mL/kg) | 7 | Once daily for 14 days |
| G6 (AJ003-36 Medium Dose) | AJ003-36 | Subcutaneous injection | 1 mg/kg (1 mL/kg) | 7 | Once daily for 14 days |
| G7 (AJ003-36 High Dose) | AJ003-36 | Subcutaneous injection | 2 mg/kg (1 mL/kg) | 7 | Once daily for 14 days |

### Example 10: Evaluation of In Vivo Analgesic Activity of Candidate Modified Peptides at Different Doses in the Mouse Diabetic Neuropathic Pain Model

Except for the control group, the remaining male C57 mice (Hangzhou Ziyuan Experimental Animal Technology Co., Ltd.) were continuously fed a high-sugar, high-fat diet for approximately ten weeks. For the model group, mice were fasted overnight, and streptozotocin (STZ) (Sann Chemical Technology (Shanghai) Co., Ltd.) was dissolved in sodium citrate-citric acid buffer solution and intraperitoneally injected at a dose of 70 mg/kg. About two weeks after STZ injection, the mechanical pain threshold of mice was measured. Mice were placed on a perforated wire mesh for adaptation, and the plantar surface of the right hind paw was vertically stimulated with a blunt needle. Pain threshold data were collected using a biosignal acquisition system. Each mouse was measured three times, and the average value was calculated. Mice were then randomly assigned to groups based on their pain thresholds for drug administration.

Day 1 of dosing: Drug administration was performed according to Table 9. Mechanical pain thresholds of the plantar surface were measured at 0 h, 2 h, 4 h, 6 h, and 8 h after administration. As shown in FIG. 26, at 2 h and 4 h, pregabalin, parent peptide AJ003, and candidate modified peptide AJ003-36 at low, medium, and high doses all exhibited significant analgesic activity compared with the model group. At 6 h and 8 h, AJ003 did not show significant differences compared with the model group, whereas pregabalin and AJ003-36 at all doses still showed significant analgesic activity.

Day 4 of dosing: Drug administration was performed according to Table 9. Mechanical pain thresholds were measured at 0 h, 2 h, 4 h, 6 h, and 8 h after administration. As shown in FIG. 27, at 2 h and 4 h, pregabalin, AJ003, and AJ003-36 at low, medium, and high doses all showed significant analgesic activity compared with the model group. At 6 h and 8 h, AJ003 showed no significant difference from the model group, whereas pregabalin and AJ003-36 at all doses still had significant analgesic activity.

Day 8 of dosing: Drug administration was performed according to Table 9. Mechanical pain thresholds were measured at 0 h, 2 h, 4 h, 6 h, and 8 h after administration. As shown in FIG. 28, at 2 h and 4 h, pregabalin, AJ003, and AJ003-36 at low, medium, and high doses all demonstrated significant analgesic activity. At 6 h and 8 h, AJ003 and AJ003-36 low-dose groups showed no significant difference compared with the model group, while pregabalin and AJ003-36 medium/high dose groups still had significant analgesic activity.

Day 14 of dosing: Drug administration was performed according to Table 9. Mechanical pain thresholds were measured at 0 h, 2 h, 4 h, 6 h, and 8 h after administration. As shown in FIG. 29, at 2 h and 4 h, pregabalin, AJ003, and AJ003-36 at low, medium, and high doses all showed significant analgesic activity compared with the model group. At 6 h and 8 h, AJ003 did not show significant differences, while pregabalin and AJ003-36 at all doses still showed significant analgesic activity.

Based on mechanical pain threshold measurements over four administrations, AJ003 showed significant analgesic activity within 4 h compared with the model group, but no significant effect beyond 4 h. Candidate modified peptide AJ003-36 at medium and high doses exhibited significant analgesic activity from 2 h to 8 h compared with the model group. The low-dose AJ003-36 group showed no significant difference on Day 8, but significant analgesic activity on the other three testing days. The high-dose AJ003-36 group displayed slightly superior analgesic activity compared with pregabalin. These results indicate that the candidate modified peptide AJ003-36, administered subcutaneously, can treat diabetic-induced neuropathic pain.

**Table 9 Dosing Scheme for Mouse Diabetic Neuropathic Pain Model**

| Group | Drug | Administration Route | Dose | Number of Animals | Dosing Frequency |
|---|---|---|---|---|---|
| G1 (Blank/Control) | Saline | Subcutaneous injection | 0 mg/kg (10 mL/kg) | 7 | Once daily for 14 days |
| G2 (Model) | Saline | Subcutaneous injection | 0 mg/kg (10 mL/kg) | 7 | Once daily for 14 days |
| G3 (Positive Drug) | Pregabalin | Oral | 60 mg/kg (6 mL/kg) | 7 | Once daily for 14 days |
| G4 (AJ003) | AJ003 | Subcutaneous injection | 2 mg/kg (10 mL/kg) | 7 | Once daily for 14 days |
| G5 (AJ003-36 Low Dose) | AJ003-36 | Subcutaneous injection | 1 mg/kg (10 mL/kg) | 7 | Once daily for 14 days |
| G6 (AJ003-36 Medium Dose) | AJ003-36 | Subcutaneous injection | 2 mg/kg (10 mL/kg) | 7 | Once daily for 14 days |
| G7 (AJ003-36 High Dose) | AJ003-36 | Subcutaneous injection | 3 mg/kg (10 mL/kg) | 7 | Once daily for 14 days |

## Claims

1. A peptide or a derivative thereof, comprising the sequence from the N-terminus to the C-terminus as shown in Formula I, or a sequence with conservative substitutions of Formula I:
Gly-Ser-Cys-Ser-X1-X2-X3-X4-(D-Cys)-X5-X6-X7-X8 Formula I,
wherein the cysteine at position 3 and D-cysteine at position 9 form an intramolecular disulfide bond in Formula I, D-Cys represents D-cysteine, the "-" between amino acid residues represents a peptide bond, the C-terminus of the peptide or its derivative is a free carboxyl group or an amide, X1 is Thr or D-Thr, X5 is selected from any one of Ala, Val, and D-Val, and X2-X4 and X6-X8 are independently any amino acid.

2. The peptide or a derivative thereof according to claim 1, wherein X2 is selected from any one of Pro, D-Pro, and Hyp; X3 is selected from any one of Pro, D-Pro, and Hyp; X4 is selected from any one of Ser, D-Ser, Ala, and Aib; X6 is selected from any one of Ala, Leu, D-Leu, Ile, ABu, and Nle; X7 is selected from any one of Tyr, D-Tyr, Ala, Trp, Phe, and Bip; and X8 is selected from any one of Ser, D-Ser, Ala, and Orn.

3. The peptide or a derivative thereof according to claim 1 or 2, comprising any one sequence selected from the following sequences: SEQ ID NOs: 1-5, SEQ ID NOs: 10-16, and SEQ ID NOs: 20-34, or a sequence thereof having conservative substitutions; preferably, the peptide or a derivative thereof comprises any one sequence selected from the following sequences: SEQ ID NOs: 26, 28, and 32, or a sequence thereof having conservative substitutions.

4. The peptide or a derivative thereof according to any one of claims 1-3, wherein the derivative comprises one or more modifications selected from the following modifications: amino acid modifications, conservative amino acid substitutions, and substitution of hydrogen atoms in amino acid residues, as compared to the peptide.

5. A pharmaceutical composition comprising the peptide or a derivative thereof according to any one of claims 1-4, and a pharmaceutically acceptable carrier or salt.

6. Use of the peptide or a derivative thereof according to any one of claims 1-4, or the pharmaceutical composition according to claim 5, in the preparation of a medicament for treating pain.

7. A method for treating pain, comprising administering to a subject in need thereof a therapeutically effective amount of the peptide or a derivative thereof according to any one of claims 1-4, or the pharmaceutical composition according to claim 5.

8. The method according to claim 7, wherein the peptide or a derivative thereof is administered via subcutaneous injection, intramuscular injection, intravenous injection, intraperitoneal injection, intrathecal injection, oral administration, transdermal administration, pulmonary administration, ocular administration, or topical administration.

9. The use according to claim 6 or the method according to claim 7, wherein the pain comprises chemotherapy-induced pain, diabetic peripheral neuropathic pain, sciatica, osteoarticular pain, postherpetic neuralgia, infection-induced pain, nutritional deficiencyinduced pain, inflammatory pain, chronic alcohol-induced pain, hypothyroidism-associated pain, autoimmune disease pain, Guillain-Barré syndrome pain, toxic pain, drug-induced pain, tumor lesion pain, genetic disorder-associated pain, and contusion pain.
